# EUROPEAN PATENT SPECIFICATION

(11) **EP 1 608 309 B1**
(45) Date of publication and mention of the grant of the patent: **11.07.2012**
(21) Application number: 04709028.7
(22) Date of filing: 06.02.2004
(51) Int. Cl.: A61K 31/5575, C07C 405/00, C07D 333/56, C07D 333/62, C07D 409/08

(54) **10,10-DIALKYL PROSTANOIC ACID DERIVATIVES AS AGENTS FOR LOWERING INTRAOCULAR PRESSURE**
10,10-DIALKYLPROSTANONSÄURE-DERIVATE ALS MITTEL ZUR SENKUNG DES AUGENINNENDRUCKS
DERIVES DE L'ACIDE 10,10-DIALKYL-PROSTANOIQUE UTILISES COMME AGENTS REDUCTEURS DE LA PRESSION INTRAOCULAIRE

(30) Priority: 11.02.2003 US 365369; 04.02.2004 US 772720
(43) Date of publication of application: 28.12.2005
(60) Divisional application: 10178294.4
(73) Proprietor: ALLERGAN, INC., Irvine CA 92612 (US)
(72) Inventor: DONDE, Yariv, Dana Point, CA 92629 (US); NGUYEN, Jeremiah, H., La Puente, CA 91744 (US)
(74) Representative: HOFFMANN EITLE
(86) International application number: PCT/US2004/003433
(87) International publication number: WO 2004/071428

(56) References cited:
- US-A- 4 117 014
- PERNET, ANDRE G. ET AL: "Prostaglandin analogs modified at the 10 and 11 positions" TETRAHEDRON LETTERS , (41), 3933-6 CODEN: TELEAY; ISSN: 0040-4039, 1979, XP000957065
- PLANTEMA, OTTO G. ET AL: "Synthesis of (.+-.)-10,10-dimethylprostaglandin E1 methyl ester and its 15-epimer" JOURNAL OF THE CHEMICAL SOCIETY, PERKIN TRANSACTIONS 1: ORGANIC AND BIO-ORGANIC CHEMISTRY (1972-1999) , (3), 304-8 CODEN: JCPRB4; ISSN: 0300-922X, 1978, XP000955879
- PLANTEMA, O. G. ET AL: "Synthesis of 10,10-dimethylprostaglandin E1" TETRAHEDRON LETTERS , (51), 4595-8 CODEN: TELEAY; ISSN: 0040-4039, 1975, XP000957113
- HAMON A ET AL: "SYNTHESIS OF (+-)- AND 15-EPI(+-)-10,10-DIMETHYLPROSTAGLANDIN E1" TETRAHEDRON LETTERS, ELSEVIER SCIENCE PUBLISHERS, AMSTERDAM, NL, no. 3, January 1976 (1976-01), pages 211-214, XP000957115 ISSN: 0040-4039
- PATENT ABSTRACTS OF JAPAN vol. 0122, no. 02 (C-503), 10 June 1988 (1988-06-10) & JP 63 002972 A (NIPPON IYAKUHIN KOGYO KK), 7 January 1988 (1988-01-07)
- PATENT ABSTRACTS OF JAPAN vol. 0082, no. 18 (C-245), 4 October 1984 (1984-10-04) & JP 59 101458 A (NIHON IYAKUHIN KOGYO KK), 12 June 1984 (1984-06-12)

## Description

### Field of the Invention

The present invention relates to prostanoic acid derivatives as potent ocular hypotensives that are particularly suited for the management of glaucoma.

### Background of the Invention

### Description of Related Art

Ocular hypotensive agents are useful in the treatment of a number of various ocular hypertensive conditions, such as post-surgical and post-laser trabeculectomy ocular hypertensive episodes, glaucoma, and as presurgical adjuncts.

Glaucoma is a disease of the eye characterized by increased intraocular pressure. On the basis of its etiology, glaucoma has been classified as primary or secondary. For example, primary glaucoma in adults (congenital glaucoma) may be either open-angle or acute or chronic angle-closure. Secondary glaucoma results from pre-existing ocular diseases such as uveitis, intraocular tumor or an enlarged cataract.

The underlying causes of primary glaucoma are not yet known. The increased intraocular tension is due to the obstruction of aqueous humor outflow. In chronic open-angle glaucoma, the anterior chamber and its anatomic structures appear normal, but drainage of the aqueous humor is impeded. In acute or chronic angle-closure glaucoma, the anterior chamber is shallow, the filtration angle is narrowed, and the iris may obstruct the trabecular meshwork at the entrance of the canal of Schlemm. Dilation of the pupil may push the root of the iris forward against the angle, and may produce pupilary block and thus precipitate an acute attack. Eyes with narrow anterior chamber angles are predisposed to acute angle-closure glaucoma attacks of various degrees of severity.

Secondary glaucoma is caused by any interference with the flow of aqueous humor from the posterior chamber into the anterior chamber and subsequently, into the canal of Schlemm. Inflammatory disease of the anterior segment may prevent aqueous escape by causing complete posterior synechia in iris bombe, and may plug the drainage channel with exudates. Other common causes are intraocular tumors, enlarged cataracts, central retinal vein occlusion, trauma to the eye, operative procedures and intraocular hemorrhage.

Considering all types together, glaucoma occurs in about 2% of all persons over the age of 40 and may be asymptotic for years before progressing to rapid loss of vision. In cases where surgery is not indicated, topical b-adrenoreceptor antagonists have traditionally been the drugs of choice for treating glaucoma.

Certain eicosanoids and their derivatives have been reported to possess ocular hypotensive activity, and have been recommended for use in glaucoma management. Eicosanoids and derivatives include numerous biologically important compounds such as prostaglandins and their derivatives. Prostaglandins can be described as derivatives of prostanoic acid which have the following structural formula:

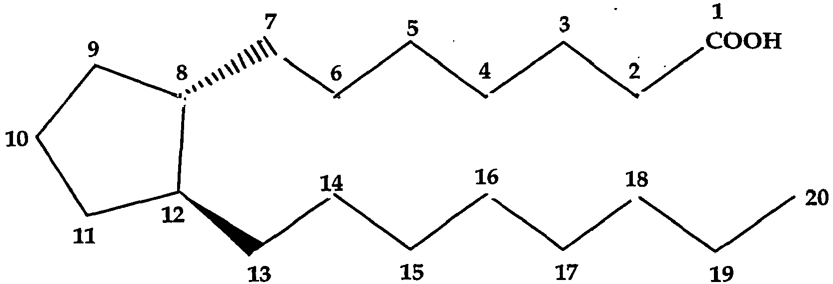

Various types of prostaglandins are known, depending on the structure and substituents carried on the alicyclic ring of the prostanoic acid skeleton. Further classification is based on the number of unsaturated bonds in the side chain indicated by numerical subscripts after the generic type of prostaglandin [e.g. prostaglandin E₁ (PGE₁), prostaglandin E₂ (PGE₂)], and on the configuration of the substituents on the alicyclic ring indicated by α or β [e.g. prostaglandin F_{2α} (PGF₂β)].

Prostaglandins were earlier regarded as potent ocular hypertensives, however, evidence accumulated in the last decade shows that some prostaglandins are highly effective ocular hypotensive agents, and are ideally suited for the long-term medical management of glaucoma (see, for example, Bito, L.Z. Biological Protection with Prostaglandins, Cohen, M.M., ed., Boca Raton, Fla, CRC Press Inc., 1985, pp. 231-252; and Bito, L.Z., Applied Pharmacology in the Medical Treatment of Glaucomas Drance, S.M. and Neufeld, A.H. eds., New York, Grune & Stratton, 1984, pp. 477-505. Such prostaglandins include PGF_{2α}, PGF_{1α}, PGE₂, and certain lipid-soluble esters, such as C₁ to C₂ alkyl esters, e.g. 1-isopropyl ester, of such compounds.

Although the precise mechanism is not yet known experimental results indicate that the prostaglandin-induced reduction in intraocular pressure results from increased uveoscleral outflow [Nilsson et. al., Invest. Ophthalmol. Vis. Sci. (suppl), 284 (1987)].

The isopropyl ester of PGF_{2α} has been shown to have significantly greater hypotensive potency than the parent compound, presumably as a result of its more effective penetration through the cornea. In 1987, this compound was described as "the most potent ocular hypotensive agent ever reported" [see, for example, Bito, L.Z., Arch. Ophthalmol. 105, 1036 (1987), and Siebold et al., Prodrug 5 3 (1989)].

Whereas prostaglandins appear to be devoid of significant intraocular side effects, ocular surface (conjunctival) hyperemia and foreign-body sensation have been consistently associated with the topical ocular use of such compounds, in particular PGF_{2α} and its prodrugs, e.g., its 1-isopropyl ester, in humans. The clinical potentials of prostaglandins in the management of conditions associated with increased ocular pressure, e.g. glaucoma are greatly limited by these side effects.

In a series of co-pending United States patent applications assigned to Allergan, Inc. prostaglandin esters with increased ocular hypotensive activity accompanied with no or substantially reduced side-effects are disclosed. The co-pending USSN 596,430 (filed 10 October 1990, now U.S. Patent 5,446,041), relates to certain 11-acyl-prostaglandins, such as 11-pivaloyl, 11-acetyl, 11-isobutyryl, 11-valeryl, and 11-isovaleryl PGF_{2α}. Intraocular pressure reducing 15-acyl prostaglandins are disclosed in the co-pending application USSN 175,476 (filed 29 December 1993). Similarly, 11,15- 9,15 and 9,11-diesters of prostaglandins, for example 11,15-dipivaloyl PGF_{2α} are known to have ocular hypotensive activity. See the co-pending patent applications USSN Nos. 385,645 (filed 07 July 1989, now U.S. Patent 4,994,274), 584,370 (filed 18 September 1990, now U.S. Patent 5,028,624) and 585,284 (filed 18 September 1990, now U.S. Patent 5,034,413). Recently, we have also shown that 17-napthyl and benzothienyl prostaglandin compounds also have ocular hypotensive activity (USSN 859,770, filed 17 May 2001).

Certain 15,15-dimethyl prostaglandins with antihypertensive, gastric acid secretion inhibition, and smooth muscle stimulant properties, are known to have improved metabolic stability. These are described by Pernet et al in US Patent 4,117,014 (filed 23 December 1976).

### Summary of the Invention

The present invention concerns a compound of Formula I 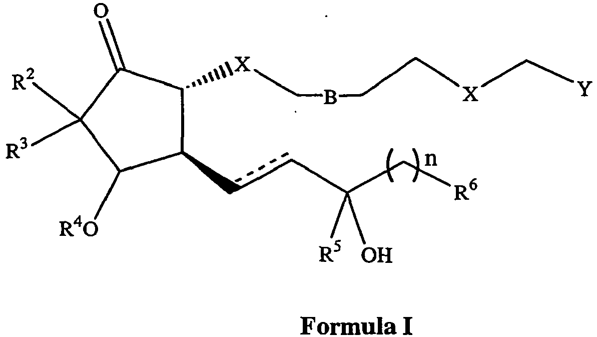 wherein the dashed line indicates the presence or absence of a bond, the hatched wedge indicates the α (down) configuration, and the solid triangle indicates the β (up) configuration;
B is a single, double, or triple covalent bond;
n is 0-6;
X is CH₂, S or O;
Y is any pharmaceutically acceptable salt of CO₂H, or CO₂R, CONR₂, CONHCH₂CH₂OH, CON(CH₂CH₂OH)₂, CH₂OR, P(O)(OR)₂, CONRSO₂R, SONR₂, or 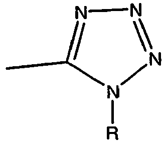 R is H, C₁₋₆ alkyl or C₂₋₆ alkenyl;
R² and R³ are C₁₋₆ linear alkyl which may be the same or different, and may be bonded to each other such that they form a ring incorporating the carbon to which they are commonly attached;
R⁴ is hydrogen, R, C(=O)R, or any group that is easily removed under physiological conditions such that R⁴ is effectively hydrogen;
R⁵ is hydrogen or R;
R⁶ is
i) hydrogen;
ii) a linear or branched hydrocarbon containing between 1 and 8 carbon atoms, which may contain one or more double or triple bonds, or oxygen or halogen derivatives of said hydrocarbon, wherein 1-3 carbon or hydrogen atoms may be substituted by O or a halogen; or
iii) aryloxy, heteroaryloxy, C₃₋₈ cycloalkyloxy, C₃₋₈ cycloalkyl, C₆₋₁₀ aryl or C₃₋₁₀ heteroaryl, wherein one or more carbons is substituted with N, O, or S; and which may contain one or more substituents selected from the group consisting of halogen, trihalomethyl, cyano, nitro, amino, hydroxy, C₆₋₁₀ aryl, C₃₋₁₀ heteroaryl, aryloxy, heteroaryloxy, C₁₋₆ alkyl, OR, SR, and SO₂R; and
the compound of Formula I is not a compound of Formula II 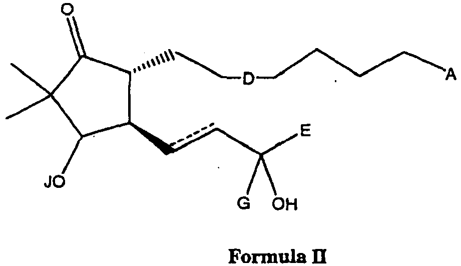 wherein A is CO₂H, CO₂Me, or CO₂Et;
D is a single, double, or triple covalent bond;
E is a linear, branched, or cycloalkyl chain of 3 to 7 carbons, trifluoromethylbutyl, hydroxylalkyl, or CH₂R⁷, wherein R⁷ is phenyl, cyclopentyl, phenoxy, chlorophenoxy, propoxy, or -CH₂SCH₂CH₃;
J is hydrogen, R, C(=O)R, or any group that is easily removed under physiological conditions such that R⁴ is effectively hydrogen; and
G is H or CH₃.

In another aspect, the present invention relates to a pharmaceutical product, comprising a container adapted to dispense its contents in a metered form; and compound therein, as hereinabove defined.

In a further aspect, certain elements of the processes of preparing the compounds represented by the above formula and described herein are novel and unobvious.

In a further aspect, the present invention relates to the above compounds for use in treating ocular hypertension or glaucoma in a mammal.

### Brief Description of the Drawing Figures

Schemes 1-8 illustrate possible ways to prepare compounds of the invention.

### Detailed Description of the Invention

The present invention relates to prostanoic acid derivative which are useful as ocular hypotensives. The compounds used in accordance with the present invention are encompassed by the following structural formula I; 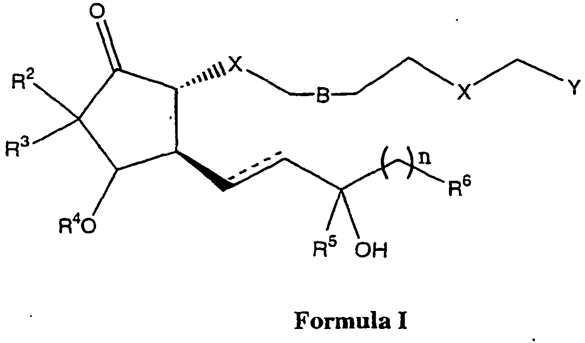 compounds that have the following structural formula II are excluded: 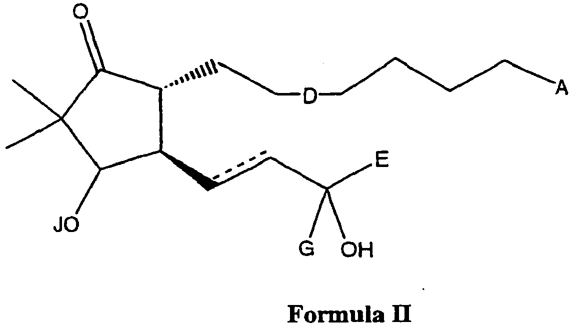 wherein A is CO₂H, CO₂Me, or CO₂Et;
D is a single, double, or triple covalent bond;
E is a linear, branched, or cycloalkyl chain of 3 to 7 carbons, trifluoromethylbutyl, hydroxylalkyl, or CH₂R⁷ wherein R⁷ is phenyl, cyclopentyl, phenoxy, chlorophenoxy, propoxy, or -CH₂SCH₂CH₃;
J is hydrogen, R, C(=O)R, or any group that is easily removed under physiological conditions such that R⁴ is effectively hydrogen; and
G is H or CH₃.

As used herein, the symbols "Me" and "Et" refer to the moieties commonly referred to as "methyl" and "ethyl" by those of ordinary skill in the art.

In other compounds related to Formula I and Formula II, A is CO₂R⁸, wherein R⁸ is any linear, branched, or cyclic alkyl group having from 3 to 6 carbons.

A preferred group includes compounds having formula III:

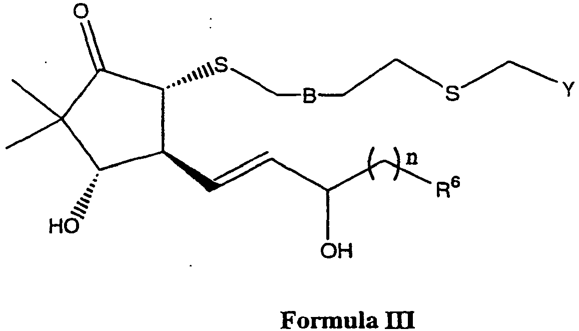

Another preferred group includes compounds having formula IV:

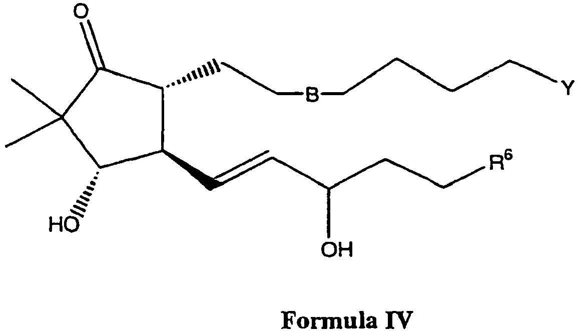

Another preferred group includes compounds having formula V: 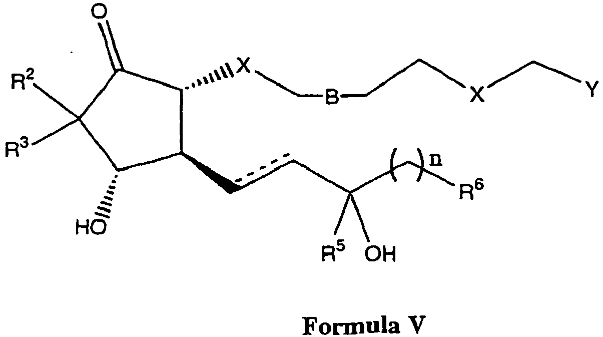 wherein at least one of R² and R³ is not methyl.

In the above formulae, the substituents and symbols are as hereinabove defined.
In the above formulae:

Preferably Y is any pharmaceutically acceptable salt of CO₂H or CO₂R. More preferably Y is CO₂H or CO₂Me.

Preferably n is 2.

Preferably, R⁶ is C₆₋₁₀ aryl or C₃₋₁₀ heteroaryl, which may contain one or more substituents selected from the group consisting of halogen, trihalomethyl, cyano, nitro, amino, hydroxy, C₁₋₆ alkyl, OR, SR, and SO₂R. More preferably R⁶ is phenyl, napthyl, benzofuranyl, or benzothienyl, which may contain one or more substituents selected from the group consisting of halogen, trihalomethyl, cyano, nitro, amino, hydroxy, C₁₋₆ alkyl, OR, SR, and SO₂R. Most preferred is 3-chlorobenzothien-2-yl.

Another preferred group includes compounds having formula XIII: 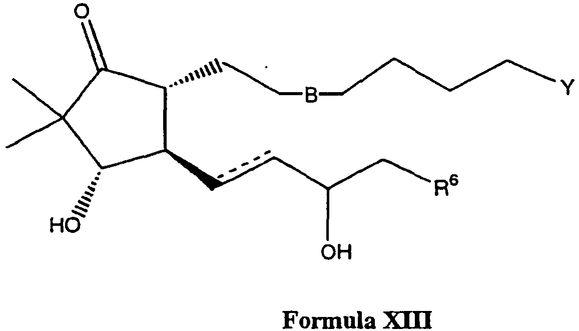 wherein B represents a single or double bond;
and R⁶ is napthyl, benzofuranyl, or benzothienyl, which may contain one or more substituents selected from the group consisting of halogen, trihalomethyl, cyano, nitro, amino, hydroxy, C₁₋₆ alkyl, OR, SR, and SO₂R.

In another aspect of this invention, certain elements of the method for making the compounds of the invention are novel and unobvious. One such novel and unobvious element is the application of the use of Baker's yeast as a reducing agent as reported by Brooks and coworkers (Brooks, et. al., "Asymmetric Microbial Reduction of Prochiral 2,2-Disubstituted Cycloalkanediones", J. Org. Chem., 1987, 52, 3223-3232) in the synthesis of compounds of this invention. Baker's yeast is used to carry out an asymmetric reduction of a compound of formula VII, which is a 2,2-diakylcyclopentane-1,3-dione, to a compound of formula VIII, which is a 2,2-dialkyl-3(S)-hydroxycyclopentanone. A compound of formula VIII is then used to prepare compounds of this invention. 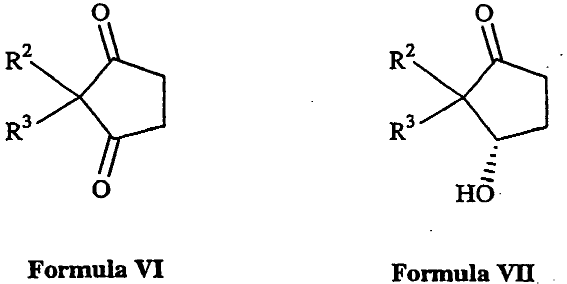 The two alkyl groups, R² and R³ of the compounds of formula VI and VII, in this reaction are the same as those defined for compounds of Formula I above. In the case where the two alkyl groups are different, a mixture of diastereomers is formed, which can be separated by conventional separation methods to obtain the enantiomerically pure products.

Preparation of 2,2-dialkylcyclopentan-1,3-diones is well known in the art. One convenient way that a large variety of these compounds can be prepared is by base-mediated alkylation of carbon-2 of the cyclopentane-1,3-dione using an alkyl halide or equivalent compound. This type of reaction is well known in the art. The preparation of three general types of 2,2-dialkylcyclopentan-1,3-diones using this alkylation reaction is illustrated in Scheme 1. Compounds where one of the alkyl groups is methyl can be prepared by a simple alkylation reaction from commercially available 2-methylcyclopentan-1,3-dione **1** (Equation 1). In the case neither of the alkyl groups in the 2,2-dialkylcyclopentan-1,3-dione are methyl (compound 2b), these compounds can be prepared from cyclopentan-1,3-dione by two consecutive alkylation reactions (Equation 2). In the case where the two alkyl groups in the 2,2-dialkylcyclopentan-1,3-dione are the same, these alkylation reactions can be carried out in a one-pot procedure. In the case where the two alkyl groups to form a cyclic compound incorporating C₂ of the cyclopentanone into the ring, otherwise known as a spiroketone, these compounds can be prepared by using a dihaloalkane or equivalent compound to carry out a intermolecular alkylation followed by an intramolecular alkylation (Equation 3), which could be carried out in a one or two pot process. Those skilled in the art will recognize that there are many ways to prepare 2,2-dialkylcyclopentan-1,3-diones, and the reactions of Scheme 1 are included to illustrate that these compounds can be readily prepared or obtained by those skilled in the art.

In another novel and unobvious aspect of this invention, compounds of the invention represented by Formula VIII are prepared by a process that comprises the following steps: 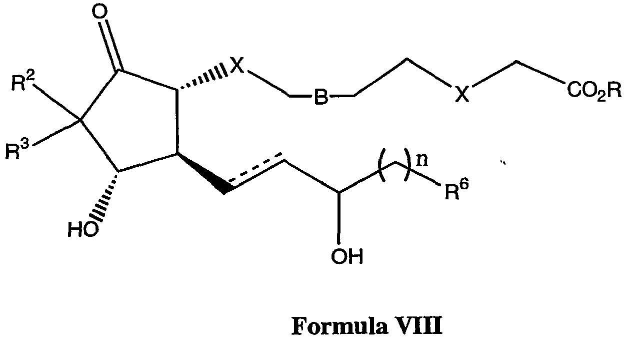
i) reacting a compound of Formula IX with a compound of Formula X in the presence of a suitable base to form a compound of Formula XI; 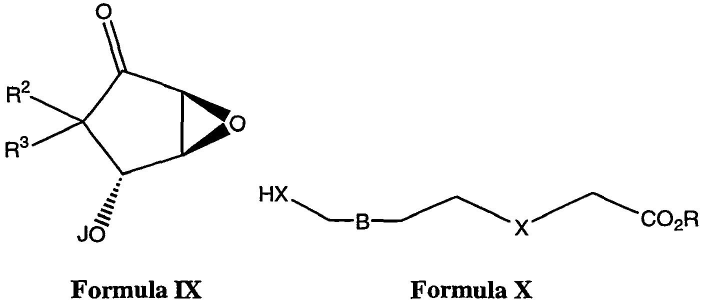 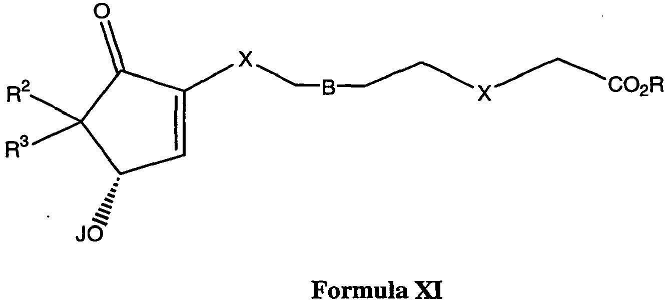
ii) coupling a compound of Formula XI with a compound of Formula XII; and 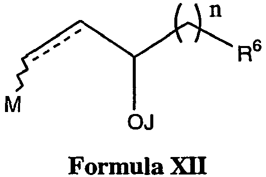
iii) removing the protecting groups and separating the diastereomers to obtain the desired products;
wherein the hatched wedges indicate the α (down) configuration, the solid triangles indicate the β (up) configuration, and the wavy lines indicate either the cis (Z) or trans (Z) conformation;
n is 0-6;
B is a single, double, or triple covalent bond;
J is a protecting group that can be easily removed to form the respective hydroxide group without affecting the rest of the molecule;
R is C₁₋₆ alkyl or C₂₋₆ alkenyl;
R² and R³ are C₁₋₆ linear alkyl which may be the same or different, and may be bonded to each other such that they form a ring incorporating the carbon to which they are commonly attached;
X is S or O; and
M is a group that comprises one or more metal atoms.

All of the compounds encompassed by this invention can be prepared using the methods described above supplemented by methods known to those skilled in the art. The synthesis of several compounds of the invention is illustrated in Schemes 2-7. These Schemes that are included herein are merely illustrative. Although there are several ways the reduction of compounds of Formula VI to Formula VII could be incorporated into the synthesis of these compounds, one convenient way to this is shown in Scheme 2. In this Scheme, compound **2** is a compound of Formula VI and compound **3** is a compound of formula VII. However, those skilled in the art will recognize that there are many ways in which the reduction could be used to prepare compounds of this invention.

**Table 1**

| Structure | Low Rf diastereomer | High Rf diastereomer |
|---|---|---|
| 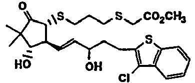 | 21 | 22 |
| 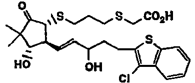 | 23 | 24 |
| 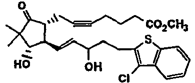 | 34 | 35 |
| 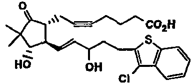 | 36 | 37 |
| 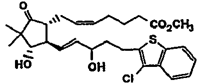 | 38 | 39 |
| 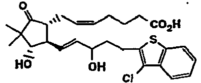 | 40 | 41 |
| 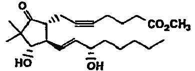 | 42 | |
| 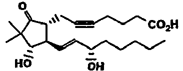 | 43 | |
| 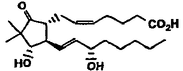 | 44 | |
| 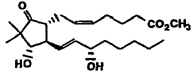 | 45 | |
| 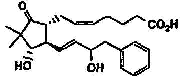 | 46 | 47 |
| 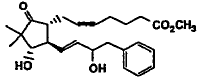 | 48 | 49 |
| 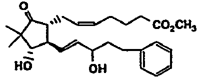 | 50 | 51 |
| 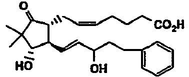 | 52 | 53 |
| 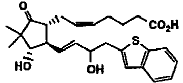 | 54 | 55 |
| 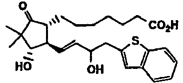 | 56 | 57 |
| 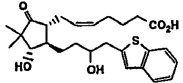 | 58 | 59 |
| 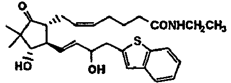 | 60 | 61 |
| 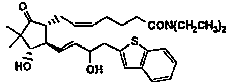 | 62 | 63 |
| 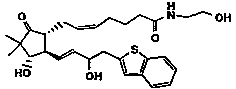 | 64 | 65 |
| 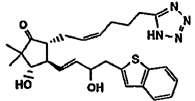 | 66 | 67 |
| 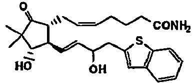 | 68 | 69 |
| 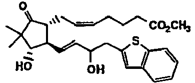 | 70 | 71 |
| 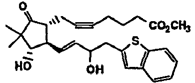 | 72 | 73 |
| 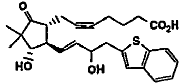 | 74 | 75 |

The compounds named below, and illustrated in Table 1, are especially preferred representatives of the compounds of the present invention:
(3-{(1*R*,4*S*,5*S*)-5-(3-chloro-benzo[b]thiophen-2-yl)-3-hydroxy-pent-1-enyl]-4-hydroxy-3,3-dimethyl-2-oxo-cyclopentylsulfanyl}-propylsulfanyl)-acetic acid methyl ester (**21, 22**);
(3-{(1*R*,4*S*,5*S*)-5-(3-chloro-benzo[b]thiophen-2-yl)-3-hydroxy-pent-1-enyl]-4-hydroxy-3,3-dimethyl-2-oxo-cyclopentylsulfanyl}-propylsulfanyl)-acetic acid (**23, 24**);
(*Z*)-7-{(1*R*,4*S*,5*R*)-5-[(*E*)-5-(3-chloro-benzo[b]thiophene-2-yl)-3-hydroxy-pent-1-enyl]-4-hydroxy-3,3-dimethyl-2-oxo-cyclopentyl}-hept-5-ynoic acid methyl ester (**34, 35**);
(*Z*)-7-{(1*R*,4*S*,5*R*)-5-[(*E*)-5-(3-chloro-benzo[b]thiophene-2-yl)-3-hydroxy-pent-1-enyl]-4-hydroxy-3,3-dimethyl-2-oxo-cyclopentyl}-hept-5-ynoic acid (**36,37**);
(*Z*)-7-{(1*R*,4*S*,5*R*)-5-[(*E*)-5-(3-chloro-benzo[b]thiophene-2-yl)-3-hydroxy-pent-1-enyl]-4-hydroxy-3,3-dimethyl-2-oxo-cyclopentyl}-hept-5-enoic acid methyl ester (**38,39**);
(*Z*)-7-{(1*R*,4*S*,5*R*)-5-[(*E*)-5-(3-chloro-benzo[b]thiophene-2-yl)-3-hydroxy-pent-1-enyl]-4-hydroxy-3,3-dimethyl-2-oxo-cyclopentyl}-hept-5-enoic acid (**40,41**);
7-[(1*R*,4*S*,5*R*)-4-Hydroxy-5-((*E*)-(S)-3-hydroxy-oct-1-enyl)-3,3-dimethyl-2-oxo-cyclopentyl]-hept-5-ynoic acid methyl ester (**42**)
7-[(1*R*,4*S*,5*R*)-4-Hydroxy-5-((*E*)-(S)-3-hydroxy-oct-1-enyl)-3,3-dimethyl-2-oxo-cyclopentyl]-hept-5-ynoic acid (**43**)
(Z)-7-[(1R,4S,5R)-4-Hydroxy-5-((E)-(S)-3-hydroxy-oct-1-enyl)-3,3-dimethyl-2-oxo-cyclopentyl]-hept-5-enoic acid (**44**)
(Z)-7-[(1R,4S,5R)-4-Hydroxy-5-((E)-(S)-3-hydroxy-oct-1-enyl)-3,3-dimethyl-2-oxo-cyclopentyl]-hept-5-enoic acid methyl ester (**45**)
(Z)-7-[(1R,4S,5R)-4-Hydroxy-5-((E)-3-hydroxy-4-phenyl-but-1-enyl)-3,3-dimethyl-2-oxo-cyclopentyl]-hept-5-enoic acid (**46, 47**)
(Z)-7-[(1R,4S,5R)-4-Hydroxy-5-((E)-3-hydroxy-4-phenyl-but-1-enyl)-3,3-dimethyl-2-oxo-cyclopentyl]-hept-5-enoic acid methyl ester (**48, 49**)
(Z)-7-[(1R,4S,5R)-4-Hydroxy-5-((E)-3-hydroxy-5-phenyl-pent-1-enyl)-3,3-dimethyl-2-oxo-cyclopentyl]-hept-5-enoic acid methyl ester (**50,51**)
(Z)-7-[(1R,4S,5R)-4-Hydroxy-5-((E)-3-hydroxy-5-phenyl-pent-1-enyl)-3,3-dimethyl-2-oxo-cyclopentyl]-hept-5-enoic acid (**52,53**)
(*Z*)-7-[(1*R*,4*S*,5*R*)-5-((*E*)-4-Benzo[b]thiophen-2-yl-3-hydroxy-but-1-enyl)-4-hydroxy-3,3-dimethyl-2-oxo-cyclopentyl]-hept-5-enoic acid (**54,55**)
7-[(1*R*,4*S*,5*R*)-5-((*E*)-4-Benzo[b]thiophen-2-yl-3-hydroxy-but-1-enyl)-4-hydroxy-3,3-dimethyl-2-oxo-cyclopentyl]-heptanoic acid (**56,57**)
(*Z*)-7-[(1*R*,4*S*,5*R*)-5-(4-Benzo[b]thiophen-2-yl-3-hydroxy-butyl)-4-hydroxy-3,3-dimethyl-2-oxo-cyclopentyl]-hept-5-enoic acid (**58,**59)
(*Z*)-7-[(1*R*,4*S*,5*R*)-5-((*E*)-4-Benzo[b]thiophen-2-yl-3-hydroxy-but-1-enyl)-4-hydroxy-3,3-dimethyl-2-oxo-cyclopentyl]-hept-5-enoic acid ethylamide (**60,61**)
(*Z*)-7-[(1*R*,4*S*,5*R*)-5-((*E*)-4-Benzo[b]thiophen-2-yl-3-hydroxy-but-1-enyl)-4-hydroxy-3,3-dimethyl-2-oxo-cyclopentyl]-hept-5-enoic acid diethylamide (**62,63**)
(*Z*)-7-[(1*R*,4*S*,5*R*)-5-((*E*)-4-Benzo[b]thiophen-2-yl-3-hydroxy-but-1-enyl)-4-hydroxy-3,3-dimethyl-2-oxo-cyclopentyl]-hept-5-enoic acid (2-hydroxy-ethyl)-amide (**64,65**)
(3*S*,4*R*,5*R*)-4-((*E*)-4-Benzo[b]thiophen-2-yl-3-hydroxy-but-1-enyl)-3-hydroxy-2,2-dimethyl-5-[(*Z*)-6-(1-H-tetrazol-5-yl)-hex-2-enyl]-cyclopentanone (**66,67**)
(*Z*)-7-[(1*R*,4*S*,5*R*)-5-((*E*)-4-Benzo[b]thiophen-2-yl-3-hydroxy-but-1-enyl)-4-hydroxy-3,3-dimethyl-2-oxo-cyclopentyl]-hept-5-enoic acid amide (**68,69**)
(2)-7-[(1*R*,4*S*,S*R*)-5-((*E*)-4-Benzo[b]thiophen-2-yl-3-hydroxy-but-1-enyl)-4-hydroxy-3,3-dimethyl-2-oxo-cyclopentyl]-hept-5-enoic acid methyl ester (**70,71**)
7-[(1*R*,4*S*,5*R*)-5-((*E*)-4-Benzo[b]thiophen-2-yl-3-hydroxy-but-1-enyl)-4-hydroxy-3,3-dimethyl-2-oxo-cyclopentyl]-hept-5-ynoic acid methyl ester (**72,73**)
7-[(1*R*,4*S*,5*R*)-5-((*E*)-4-Benzo[b]thiophen-2-yl-3-hydroxy-but-1-enyl)-4-hydroxy-3,3-dimethyl-2-oxo-cyclopentyl]-hept-5-ynoic acid (**74,75**)

Pharmaceutical compositions may be prepared by combining a therapeutically effective amount of at least one compound according to the present invention, or a pharmaceutically acceptable acid addition salt thereof, as an active ingredient, with conventional ophthalmically acceptable pharmaceutical excipients, and by preparation of unit dosage forms suitable for topical ocular use. The therapeutically efficient amount typically is between about 0.0001 and about 5% (w/v), preferably about 0.001 to about 1.0% (w/v) in liquid formulations.

For ophthalmic application, preferably solutions are prepared using a physiological saline solution as a major vehicle. The pH of such ophthalmic solutions should preferably be maintained between 6.5 and 7.2 with an appropriate buffer system. The formulations may also contain conventional, pharmaceutically acceptable preservatives, stabilizers and surfactants.

Preferred preservatives that may be used in the pharmaceutical compositions of the present invention include, but are not limited to, benzalkonium chloride, chlorobutanol, thimerosal, phenylmercuric acetate and phenylmercuric nitrate. A preferred surfactant is, for example, Tween 80. Likewise, various preferred vehicles may be used in the ophthalmic preparations of the present invention. These vehicles include, but are not limited to, polyvinyl alcohol, povidone, hydroxypropyl methyl cellulose, poloxamers, carboxymethyl cellulose, hydroxyethyl cellulose and purified water.

Tonicity adjustors may be added as needed or convenient. They include, but are not limited to, salts, particularly sodium chloride, potassium chloride, mannitol and glycerin, or any other suitable ophthalmically acceptable tonicity adjustor.

Various buffers and means for adjusting pH may be used so long as the resulting preparation is ophthalmically acceptable. Accordingly, buffers include acetate buffers, citrate buffers, phosphate buffers and borate buffers. Acids or bases may be used to adjust the pH of these formulations as needed.

In a similar vein, an ophthalmically acceptable antioxidant for use in the present invention includes, but is not limited to, sodium metabisulfite, sodium thiosulfate, acetylcysteine, butylated hydroxyanisole and butylated hydroxytoluene.

Other excipient components which may be included in the ophthalmic preparations are chelating agents. The preferred chelating agent is edentate disodium, although other chelating agents may also be used in place or in conjunction with it.

The ingredients are usually used in the following amounts:

| **Ingredient** | **Amount (% w/v)** |
|---|---|
| active ingredient | about 0.001-5 |
| preservative | 0-0.10 |
| vehicle | 0-40 |
| tonicity adjustor | 1-10 |
| buffer | 0.01-10 |
| pH adjustor | q.s. pH 4.5-7.5 |
| antioxidant | as needed |
| surfactant | as needed |
| purified water | as needed to make 100% |

The actual dose of the active compounds of the present invention depends on the specific compound, and on the condition to be treated; the selection of the appropriate dose is well within the knowledge of the skilled artisan.

The ophthalmic formulations of the present invention are conveniently packaged in forms suitable for metered application, such as in containers equipped with a dropper, to facilitate the application to the eye. Containers suitable for dropwise application are usually made of suitable inert, non-toxic plastic material, and generally contain between about 0.5 and about 15 ml solution.

### Synthetic Examples

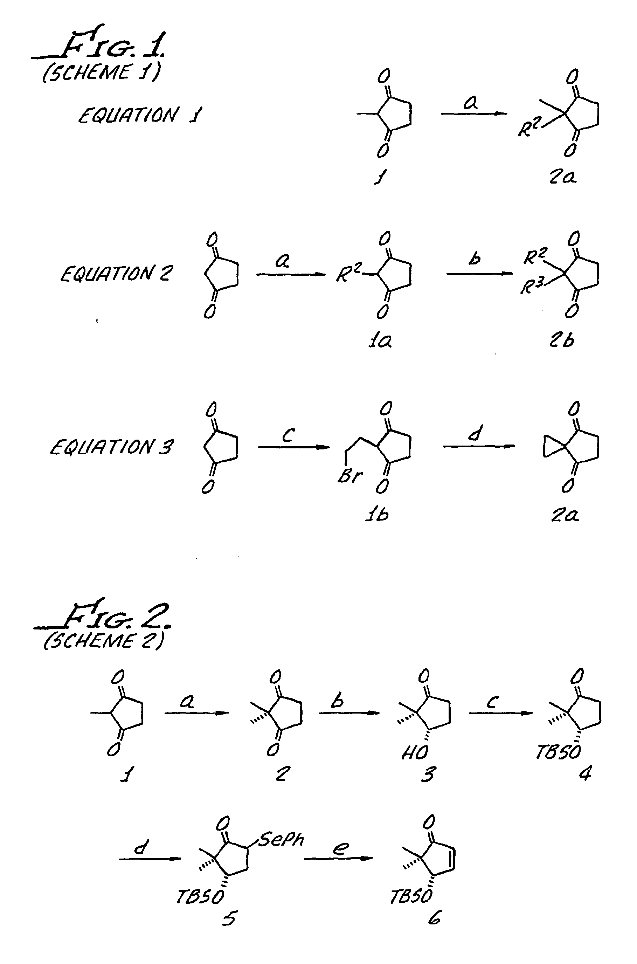

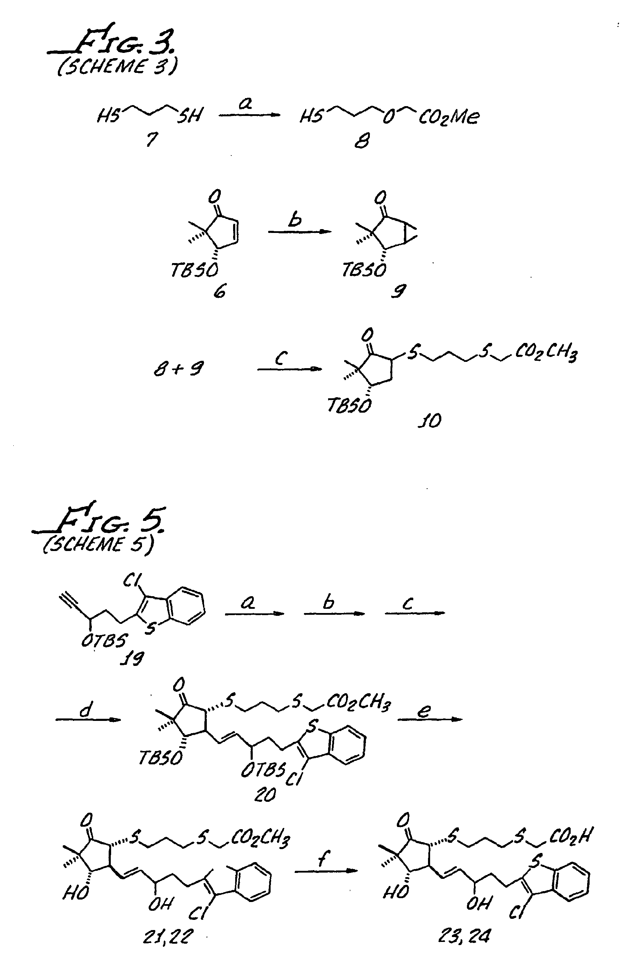

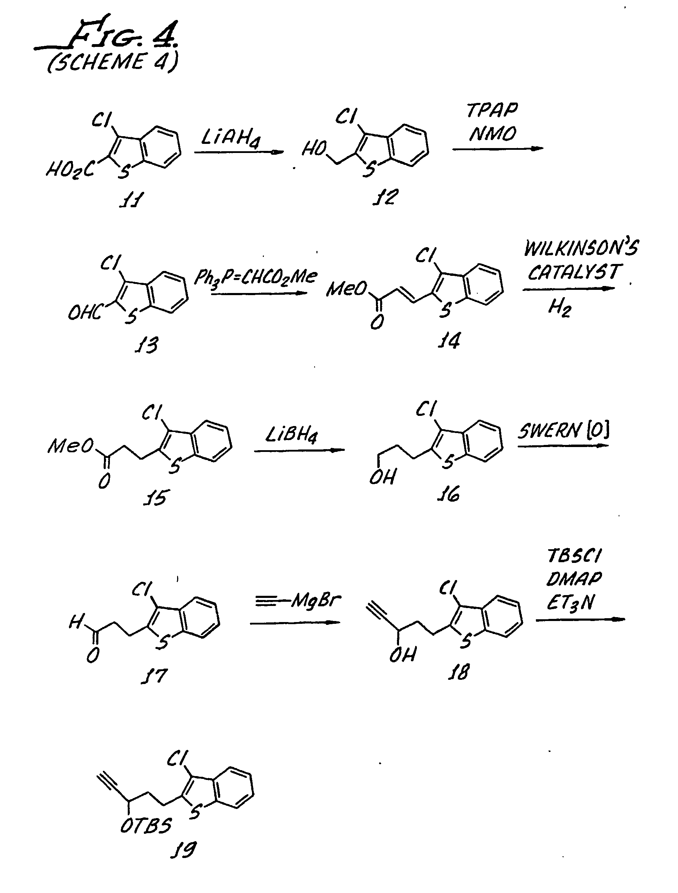

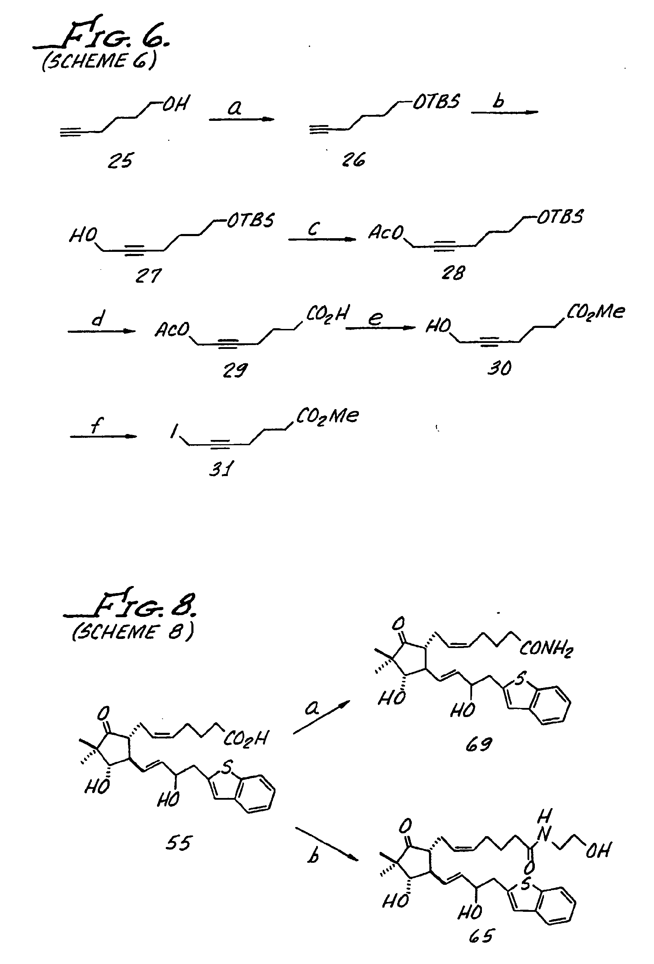

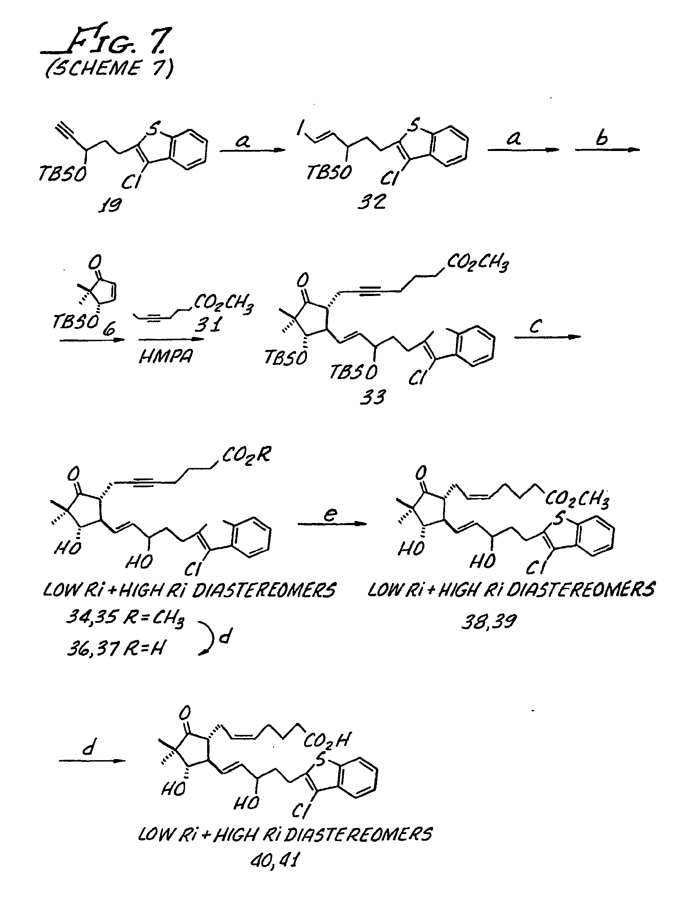

The methods of preparing compounds of this invention are further illustrated by the following non-limiting Examples, which are summarized in the reaction schemes of Figures 1-7 wherein the compounds are identified by the same designator in both the Examples and the Figures.

**2-Alkyl-cyclopentane-1,3-dione (1a)**. A mixture of 1,3-cyclopentanedione (89.4 mmol, Aldrich), I-R² (96.4 mmol, Aldrich), and KOH (5.097 g, 90.8 mmol) in H₂O (25 mL)/ dioxane (75 mL) is heated at reflux. After 5 h, a solution of KOH (2 g) and I-R² (2 mmol) in H₂O (5 mL)/ dioxane (15 mL) is added and after another 3 h at reflux the solution is allowed to stir at room temperature overnight. In the morning, the reaction is continued by addition of a solution of KOH (2 g) and I-R² (2.4 mmol) in H₂O (5 mL)/dioxane (15 mL) and heating at reflux. After 4 h, the mixture is allowed to cool to room temperature and is extracted with ether (1 x 100mL, 3 x 75 mL). The combined ether extracts are evaporated, the residue is combined with HCl (50 mL 10%), and the resulting mixture is placed in a 120 °C oil bath until boiling is observed (ca. 15 min.). The mixture is then allowed to cool to room temperature, is neutralized by addition of NaHCO₃ solution (150 mL, saturated) and the resulting mixture is then extracted with CH₂Cl₂ (4 x 75 mL). The combined CH₂Cl₂ solution is dried (MgSO₄), filtered and evaporated to leave a brown oil which is used directly in the next step.

**2-Alkyl-2-methyl-cyclopentane-1,3-dione (2a)**. A mixture of 2-methyl-1,3-cyclopentanedione (10.025 g, 89.4 mmol, Aldrich), I-R² (96.4 mmol, Aldrich), and KOH (5.097 g, 90.8 mmol) in H₂O (25 mL)/ dioxane (75 mL) is heated at reflux. After 5 h, a solution of KOH (2 g) and I-R² (2 mmol) in H₂O (5 mL)/ dioxane (15 mL) is added and after another 3 h at reflux the solution is allowed to stir at room temperature overnight. In the morning, the reaction is continued by addition of a solution of KOH (2 g) and I-R² (2.4 mmol) in H₂O (5 mL)/ dioxane (15 mL) and heating at reflux. After 4 h, the mixture is allowed to cool to room temperature and is extracted with ether (1 x 100mL, 3 x 75 mL). The combined ether extracts are evaporated, the residue is combined with HCl (50 mL 10%), and the resulting mixture is placed in a 120 °C oil bath until boiling is observed (ca. 15 min.). The mixture is then allowed to cool to room temperature, is neutralized by addition of NaHCO₃ solution (150 mL, saturated) and the resulting mixture is then extracted with CH₂Cl₂ (4 x 75 mL). The combined CH₂Cl₂ solution is dried (MgSO₄), filtered and evaporated to leave a brown oil which is used directly in the next step.

**2,2-Dialkyl-methyl-cyclopentane-1,3-dione (2b)**. A mixture of 2-alkyl-1,3-cyclopentanedione **1a** (89.4 mmol, Aldrich), I-R³ (96.4 mmol, Aldrich), and KOH (5.097 g, 90.8 mmol) in H₂O (25 mL)/ dioxane (75 mL) is heated at reflux. After 5 h, a solution of KOH (2 g) and I-R³ (2 mmol) in H₂O (5 mL)/ dioxane (15 mL) is added and after another 3 h at reflux the solution is allowed to stir at room temperature overnight. In the morning, the reaction is continued by addition of a solution of KOH (2 g) and I-R³ (2.4 mmol) in H₂O (5 mL)/ dioxane (15 mL) and heating at reflux. After 4 h, the mixture is allowed to cool to room temperature and is extracted with ether (1 x 100 mL, 3 x 75 mL). The combined ether extracts are evaporated, the residue is combined with HCl (50 mL 10%), and the resulting mixture is placed in a 120 °C oil bath until boiling is observed (ca. 15 min.). The mixture is then allowed to cool to room temperature, is neutralized by addition of NaHCO₃ solution (150 mL, saturated) and the resulting mixture is then extracted with CH₂Cl₂ (4 x 75 mL). The combined CH₂Cl₂ solution is dried (MgSO₄), filtered and evaporated to leave a brown oil which is used directly in the next step.

**Spiro[2.4]heptane-4,7-dione (2c)**. A mixture of 2-alkyl-1,3-cyclopentanedione **1a** (89.4 mmol, Aldrich), 1,2-dibromoethane (120 mmol, Aldrich), and KOH (5.097 g, 90.8 mmol) in H₂O (25 mL)/ dioxane (75 mL) is heated at reflux for 24 hours. The mixture is allowed to cool, and the crude product is extracted with ether (1 x 100mL, 3 x 75 mL). The combined ether extracts are evaporated, the residue is combined with HCl (50 mL 10%), and the resulting mixture is placed in a 120 °C oil bath until boiling is observed (ca. 15 min.). The mixture is then allowed to cool to room temperature, is neutralized by addition of NaHCO₃ solution (150 mL, saturated) and the resulting mixture is then extracted with CH₂Cl₂ (4 x 75 mL). The combined CH₂Cl₂ solution is dried (MgSO₄), filtered and evaporated to leave a brown oil which is used directly in the next step.

**2,2-Dimethyl-cyclopentane-1,3-dione (2)**. The published procedure was followed. (Agosta, W.C.; Smith, A.B. J.Org.Chem. 1970, 35, 3856) A mixture of 2-methyl-1,3-cyclopentanedione (10.025 g, 89.4 mmol, Aldrich), methyl iodide (6.0 mL, 96.4 mmol, Aldrich), and KOH (5.097 g, 90.8 mmol) in H₂O (25 mL)/ dioxane (75 mL) was heated at reflux. After 5 h, a solution of KOH (2 g) and MeI (2.4 mL) in H₂O (5 mL)/ dioxane (15 mL) was added and after another 3 h at reflux the solution was allowed to stir at room temperature overnight. In the morning, the reaction was continued by addition of a solution of KOH (2 g) and MeI (2.4 mL) in H₂O (5 mL)/ dioxane (15 mL) and heating at reflux. After 4 h, the mixture was allowed to cool to room temperature and was extracted with ether (1 x 100mL, 3 x 75 mL). The combined ether extracts were evaporated, the residue combined with HCl (50 mL 10%), and the resulting mixture was placed in a 120 °C oil bath until boiling was observed (ca. 15 min.). The mixture was then allowed to cool to room temperature, was neutralized by addition of NaHCO₃ solution (150 mL, saturated) and the resulting mixture then extracted with CH₂Cl₂ (4 x 75 mL). The combined CH₂Cl₂ solution was dried (MgSO₄), filtered and evaporated to leave a brown oil (10.474 g, 83 mmol, 93%) which was used directly in the next step. **(S)-3-Hydroxy-2,2-dimethyl-cyclopentanone (3)**. The published procedure was followed. (Brooks, D.W.; Hormoz, M.; Grothaus, P.G. J. Org. Chem. 1987, 52, 3223) A 35 °C (internal temperature) solution of D-glucose (106.73 g, 592 mmol, Aldrich) in H₂O (690 mL) in a 4 L Erlenmeyer was treated with baker's yeast (71.065 g, Fleischmann's). The mixture was allowed to ferment for 2 h, then 2,2-dimethyl-cyclopentane-1,3-dione (**2**) (7.316 g, 58 mmol) was added.

The mixture was stirred for 48 h and then filtered through celite, washing with about 1 L CH₂Cl₂. The filtration was difficult due to the thick consistency of the yeast and it helped to continually add CH₂Cl₂ to the mixture and scrape the top of the celite layer with a spatula. The filtrate was transferred to a separatory funnel, and 100 mL brine was added and the layers were separated. Brine (400 mL) was added to the aqueous layer and the resulting solution extracted further with CH₂Cl₂ (3 x 500 mL). The combined CH₂Cl₂ solution was dried (MgSO₄), filtered and evaporated to leave a yellow oil. Flash chromatography (11 x 5 cm, 20% EtOAc/hexs → 25% → 30% → 40% → 50%) gave alcohol **3** (2.435 g, 19 mmol, 33%).

The enantiomeric excess of **3** was assayed by ¹H NMR of the corresponding Mosher's ester which was prepared by treatment of alcohol **3 (11** mg, 0.09 mmol) in dichloroethane (0.3 mL, Aldrich) with pyridine (27 µL, 0.33 mmol, Aldrich) and (*R*)-α-methoxy-α-trifluoromethyphenylacetic acid chloride (58 µL, 0.31 mmol, Fluka). The mixture was stirred overnight and then partitioned between water (10 mL) and ether (10 mL). The ether layer was washed with 1 M HCl (10 mL) and saturated NaHCO₃ solution and then was dried (MgSO₄), filtered and evaporated. ¹H NMR analysis was done on the crude ester.
**(*S*)-3-(*tert*)-Butyl-dimethyl-silanyloxy-2,2-dimethyl-cyclopentanone (4)**. A solution of alcohol **3** (520 mg, 4.1 mmol) and 2,6-lutidine (0.56 mL, 4.8 mmol, Aldrich) in CH₂Cl₂ (8.0 mL, Aldrich) was treated with TBSOTf (1.0 mL, 4.3 mmol, Aldrich). After 5.5 h, saturated NaHCO₃ solution (20 mL) was added and the mixture extracted with CH₂Cl₂ (20 mL). The CH₂Cl₂ solution was washed with 20 mL each of 1 M HCl, saturated NaHCO₃ solution, and brine and then was dried (MgSO₄), filtered and evaporated. Flash chromatography (5 x 5 cm, 10% Et₂O/pentane) gave TBS ether **4** (698 mg, 2.9 mmol, 70%).
**(*S*)-3-(*tert*)-Butyl-dimethyl-silanyloxy-2,2-dimethyl-5-phenylselanyl-cyclopentanone (5)**. A solution of TBS ether **4** (1.496 g, 6.2 mmol) in THF (2 mL, Aldrich) was added dropwise to a -78 °C solution of LDA (4.9 mL, 7.3 mmol, 1.5 M/cyclohexane, Aldrich) in THF (22 mL, Aldrich), rinsing with 2 mL THF. After 15 min., a solution of PhSeCl (1.424 g, 7.4 mmol, Aldrich) in THF (2 mL) was quickly added by cannula, rinsing with 2 mL THF. The solution was stirred for 10 min. and then partitioned between 50 mL 0.5 M HCl and 75 mL ether. The ether layer was washed with 30 mL each of water, saturated NaHCO₃ solution, and brine and then was dried (MgSO₄), filtered and evaporated. Flash chromatography (2% EtOAc/hexs → 4%) gave phenylselenide **5** (1.641 g, 4.1 mmol, 67%) along with 476 mg of mixed fractions containing a lower R_{f} impurity.
**(*S*)-4-(*tert*)-Butyl-dimethyl-silanyloxy-5,5-dimethyl-cyclopent-2-enone(6).** A solution of selenide **5** (1.641 g, 4.1 mmol) and pyridine (0.62 mL, 7.7 mmol, Aldrich) in CH₂Cl₂ (13 mL, Aldrich) was treated with H₂O (1 mL) and 30% H₂O₂ (1.1 mL, Aldrich). The mixture was stirred for 30 min. and then was partitioned between 25 mL CH₂Cl₂ and 25 mL saturated NaHCO₃ solution. The aqueous layer was extracted with 25 mL CH₂Cl₂ and the combined CH₂Cl₂ solution washed with 1 M HCl (2 x 25 mL) and brine (50 mL). The solution was then dried (MgSO₄), filtered and evaporated to leave an orange oil. Flash chromatography (6 x 4 cm, 10% ether/pentane) gave enone **6** (572 mg, 2.4 mmol, 59%).
**(3-Mercapto-propylsulfanyl)-acetic acid methyl ester (8)**. An ice-cold solution of 1,3-dithiane (2.0 mL, 19.9 mmol) in THF (40 mL) was treated with NaH (819 mg, 20.5 mmol). After 30 min., methyl bromoacetate (1.9 mL, 20.0 mmol) was added and the mixture stirred for 3.5 h at room temperature. The reaction was quenched by addition of MeOH and then 50 mL 1 M HCl. The mixture was extracted with ether (2 x 50 mL) and the combined ether solution washed with saturated sodium bicarbonate solution (50 mL) and brine (50 mL) and then was dried (MgSO₄), filtered and evaporated. Purification by flash chromatography on silica gel (10-15% ethyl acetate/hexanes) gave 971 mg (5.38 mmol, 27 %) of the thiol.
**{3-[(*S*)-3-(*tert*)-Butyl-dimethyl-silanyloxy)-4,4-dimethyl-5-oxo-cyclopent-1-enylsulfanyl]-propylsulfanyl}-acetic acid methyl ester (10)**. A solution of enone **6** (156 mg, 0.65 mmol) in MeOH (4.3 mL) was treated with 30 % H₂O₂ (0.21 mL) and 1 M NaOH (32 µL). After 4 h, 20 mL saturated ammonium chloride solution was added and the mixture was extracted with dichloromethane (3 x 10 mL). The combined dichloromethane solution was dried (Na₂SO₄), filtered and evaporated in vacuo.

A solution of thiol **8** (110 mg, 0.61 mmol) in dichloromethane (3 mL) was added to the crude epoxide (**9**) by cannula, rinsing with 1.2 mL. Basic alumina (628 mg) was added and the mixture stirred for 16 h. The solvent was evaporated and purification of the residue by flash chromatography on silica gel (15% ethyl acetate/hexanes) gave 129 mg (0.31 mmol, 48 %) of the coupled enone (**10**).
**(3-Chloro-benzo[b]thiophen-2-yl)-methanol (12)**. To an ice cold solution of 10.0 g (47.0 mmol) of 3-chloro-benzo[b]thiophene-2-carboxylic acid (**11**) in 200 mL of THF was added 47 mL of LiAlH₄ (47 mmol, 1 M/THF). After 3 h, the reaction was quenched by addition of MeOH (ca. 40 mL). The volatiles were evaporated and the residue was treated with 50 mL 1 M HCl. After stirring for 10 min., the mixture was extracted with CH₂Cl₂ (3 x 150 mL). The combined CH₂Cl₂ solution was dried (MgSO₄), filtered and evaporated. Purification by flash chromatography on silica gel (10-20% ethyl acetate/hexane) gave 4.32 g (21.6 mmol, 46 %) of the alcohol (**12**).
**3-Chloro-benzo[b]thiophene-2-carbaldehyde (13)**. A solution of alcohol **12** (4.32 g, 21.6 mmol) in 40 mL of CH₂Cl₂ was treated with 4A molecular sieves, NMO (3.81 g, 32.5 mmol), and TPAP (381 mg, 1.08 mmol). The reaction was stirred for 10 min. and then was evaporated to dryness. Purification by flash chromatography on silica gel (2% ethyl acetate/hexane) gave 3.52 g (18.3 mmol, 84%) of the aldehyde (**13**).
**(*E*)-3-(3-Chloro-benzo[b]thiophen-2-yl)-acrylic acid methyl ester (14)**. A solution of 3.52 g (18.3 mmol) of **13** in 50 mL toluene was treated with methyl(triphenylphosphoranylidene)acetate (7.48 g, 21.9 mmol). After 4 h, saturated NaHCO₃ solution (50 mL) was added and the mixture extracted with ethyl acetate (2 x 75 mL). The combined ethyl acetate solution was washed with brine (50 mL), dried (Na₂SO₄), filtered and evaporated. Purification by flash chromatography on silica gel (5% ethyl acetate/hexane) provided 3.60 g (14.6 mmol, 80%) of the enoate (**14**).
**3-(3-Chloro-benzo[b]thiophen-2-yl)-propionic acid methyl ester (15)**. A solution of 3.60 g (14.6 mmol) of **14** in 50 mL THF was treated with Wilkinson's catalyst (3.35 g, 3.62 mmol). The mixture was stirred under 1 atm H₂ for 18 h and then was filtered through celite. The solvent was evaporated and the residue purified by flash chromatography on silica gel (0-2% ethyl acetate/hexane) to give 3.63 g (14.3 mmol, 99%) of the saturated ester (**15**).
**3-(3-Chloro-benzo[b]thiophen-2-yl)-propan-1-ol (16)**. An ice cold solution of 3.63 g (14.3 mmol) of **15** in 60 mL of ether was treated with LiBH₄ (621 mg, 28.5 mmol) and methanol (2 mL). After 30 min., 30 mL of 0.5 M NaOH solution was added. The mixture was extracted with ethyl acetate (2 x 25 mL) and the combined ethyl acetate solution washed with brine (50 mL), dried (MgSO₄), filtered and evaporated. The residue was purified by flash chromatography on silica gel (5-20% ethyl acetate/hexane) to give 2.57 g (11.3 mmol, 79%) of the alcohol (**16**).
**3-(3-Chloro-benzo[b]thiophen-2-yl)-propionaldehyde (17)**. A -78 °C solution of oxalyl chloride (1.73 g, 13.6 mmol) in dichloromethane (20 mL) was treated with DMSO (20 mL). After 5 min., a solution of alcohol **16** (2.57g, 11.3 mmol) in dichloromethane (20 mL) was added. After another 15 min., triethylamine (7.1 mL, 50.6 mmol) was added. The reaction was stirred at -78 °C for 5 min., and then allowed to warm to room temperature. After 30 min., 100 mL water was added and the mixture extracted with dichloromethane (3 x 60 mL). The combined dichloromethane solution was dried (Na₂SO₄), filtered and evaporated. Purification by flash chromatography on silica gel (10% ethyl acetate/hexane) gave 2.11 g (9.4 mmol, 83%) of the aldehyde (**17**).
**5-(3-Chloro-benzo[b]thiophen-2-yl)-pent-1-yn-3-ol (18)**. A solution of aldehyde **17** (2.11 g, 9.4 mmol) in 15 mL THF was added to a solution of ethynylmagnesium bromide (28.2 mL, 14.1 mmol, 0.5 M THF) at 0 °C. After 1.5 h, saturated NH₄Cl solution (75mL) was added and the mixture was extracted with ethyl acetate (3 x 50 mL). The combined ethyl acetate solution was washed with brine (50 mL) and then was dried (Na₂SO₄), filtered and evaporated. Purification by flash chromatography (5-20% ethyl acetate/hexane) gave 2.20 g (8.78 mmol, 93%) of the alcohol (**18**).
***tert*-Butyl-{1-[2-(3-chloro-benzo[b]thiophen-2-yl)-ethyl]-prop-2-ynyloxy}-dimethyl-silane (19)**. A solution of alcohol **18** (2.20 g, 8.78 mmol) in dichloromethane (15 mL) was treated with DMAP (215 mg, 1.8 mmol), TBSCI (1.59 g, 10.5 mmol), and triethylamine (1.8 mL, 13.2 mmol). The reaction was stirred for 24 h and then saturated sodium bicarbonate solution (50 mL) was added. The mixture was extracted with dichloromethane (2 x 50 mL) and the combined dichloromethane solution dried (Na₂SO₄), filtered and evaporated. Purification by flash chromatography (4% ethyl acetate/hexane) gave 3.06 g (6.4 mmol, 73%) of the protected alcohol (**19**).
**(3-{(1*R*,4S,5*S*)-4-(*tert*-Butyl-dimethyl-silanyloxy)-5-[(*E*)-3-(*tert*-butyl-dimethyl-silanyloxy)-5-(3-chloro-benzo[b]thiophen-2-yl)-pent-1-enyl]-3,3-dimethyl-2-oxo-cyclopentylsulfanyl}-propylsulfanyl)-acetic acid methyl ester (20)**. A solution of alkyne **19** (105 mg, 0.28 mmol) in THF (1.2 mL) was treated with bis(cyclopentadienyl)zirconium chloride hydride (91 mg, 0.35 mmol). The reaction was stirred for 30 min., then was cooled to -78 °C and treated with methyllithium (0.46 mL, 0.64 mmol, 1.4 M in ether). After 10 min., a precooled (-78 °C) solution of lithium 2-thienylcyanocuprate (1.3 mL, 0.33 mmol, 0.25 M in THF) was added by cannula. The reaction was stirred for 45 min. and then enone **10** (61 mg, 0.15 mmol) in 0.2 mL THF was added by cannula, rinsing with 0.2 mL THF. After 1 h, The reaction was quenched by addition of 20 mL 1:1 saturated ammonium chloride solution/concentrated ammonium hydroxide. The mixture was stirred for 45 min. and then was extracted with ethyl acetate (3 x 20 mL). The combined ethyl acetate solution was dried (Na₂SO₄), filtered and evaporated. Purification by flash chromatography on silica gel (10 % ethyl acetate/hexanes) gave 51 mg (0.064 mmol, 43%) of the coupled product (**20**).
**(3-{(1*R*,4*S*,5*S*)-5-(3-chloro-benzo[b]thiophen-2-yl)-3-hydroxy-pent-1-enyl]-4-hydroxy-3,3-dimethyl-2-oxo-cyclopentylsulfanyl}-propylsulfanyl)-acetic acid methyl ester (21, 22)**. A solution of **20** (51 mg, 0.064 mmol) in CH₃CN (1.6 mL) was treated with HF-pyridine (0.26 mL). The reaction was stirred for 24 h and then was quenched by addition of 15 mL saturated sodium bicarbonate solution. The mixture was extracted with dichloromethane (3 x 10 mL) and the combined dichloromethane solution was dried (Na₂SO₄), filtered and evaporated. Purification by preparative thin layer chromatography on silica gel (40% ethyl acetate/hexanes) gave 12 mg (0.023 mmol, 71 %) of each diastereomer.
**(3-{(1*R*,4*S*,5*S*)-5-(3-chloro-benzo[b]thiophen-2-yl)-3-hydroxy-pent-1-enyl]-4-hydroxy-3,3-dimethyl-2-oxo-cyclopentylsulfanyl}-propylsulfanyl)-acetic acid (23, 24).** Rabbit liver esterase (9 mg) was added to a solution of the lower R_{f} ester **21** (11 mg, 0.021 mmol) in pH 7.2 phosphate buffer (0.5 mL)/CH₃CN (0.1 mL). The mixture was stirred overnight and then 10 mL 0.5 M HCl was added along with a few mL's of brine. The mixture was extracted with ethyl acetate (3 x 10 mL) and the combined ethyl acetate solution dried (Na₂SO₄), filtered and evaporated. Purification by flash chromatography on silica gel (3-5% MeOH/CH₂Cl₂) gave 4 mg (0.0078 mmol, 37%) of the acid (23). 300 MHz ¹H NMR (CDCl₃, ppm) δ 7.73 (2 H, d, J = 8.4 Hz) 7.4-7.3 (2 H, m) 5.9-5.8 (1 H, m) 5.8-5.7 (1 H, m) 4.4-4.3 (1 H, m) 3.63 (1 H, d, J = 9.7 Hz) 3.21 (2 H, s) 3.1-2.4 (11 H, overlapping m) 2.1-1.7 (4 H, overlapping m) 1.12 (3 H, s) 1.03 (3 H, s).

The higher R_{f} ester was hydrolyzed similarly except a solution of rabbit liver esterase (10 mg) in 0.5 mL of pH 7.2 phosphate buffer was added to a solution of the ester (10 mg, 0.019 mmol) in CH₃CN (0.2 mL). The reaction was stirred for 22 h and then worked up and purified as above. This gave 7 mg (0.013 mmol, 71 %) of the acid (**24**). 300 MHz ¹H NMR (CDCl₃, ppm) δ 7.73 (2 H, d, J = 8.8 Hz) 7.44-7.31 (2 H, m) 5.9-5.8 (1 H, m) 5.8-5.7 (1 H, m) 4.4-4.3 (1 H, m) 3.64 (1 H, d, J = 9.7 Hz) 3.3-2.3 (13 H, overlapping m) 2.1-1.7 (4 H, overlapping m) 1.12 (3 H, s) 1.03 (3 H, s)*.*
***tert*-Butyl-hex-5-ynyloxy-dimethyl-silane (26).**
**7-(*tert*-Butyl-dimethyl-silanyloxy)-hept-2-yn-1-ol (27).**
**Acetic acid 7-(*tert*-butyl-dimethyl-silanyloxy)-hept-2-ynyl ester (28).** A solution of 7-(*tert*-Butyl-dimethyl-silanyloxy)-hept-2-yn-1-ol **27** (4.507 g, 21 mmol) in pyridine (20 mL) was treated with acetic anhydride (3.0 mL, 31.8 mmol). After 18 h, the solvent was evaporated and the residue co-evaporated with toluene. The residue was used directly in the next step.
**7-Acetoxy-hept-5-ynoic acid (29)**. A solution of crude **28** in acetone (100 mL) was treated with Jones Reagent (18.0 mL, 41.4 mmol, 2.3 M). The mixture became warm and so was cooled with an ice bath. After 1 h at room temperature, 10 mL isopropyl alcohol was added and the mixture stirred further for 15 min. The mixture still had a brown color so another 10 mL isopropyl alcohol was added. After another 15 min., the color had not changed so the mixture was filtered through celite and the filtrate evaporated in vacuo. The residue was partitioned between 100 mL ether and 100 mL saturated ammonium chloride solution. The aqueous layer was extracted with 100 mL ether and the combined ether solution washed with brine and then was dried (MgSO₄), filtered and evaporated to leave a yellow oil (6.333 g) that was used directly in the next step.
**7-Hydroxy-hept-5-ynoic acid methyl ester (30)**. The crude acid **29** (6.333 g) was treated with a 1% solution of acetyl chloride in methanol (60 mL). After 16 h, sodium bicarbonate (1.966 g, 23.4 mmol) was added. The mixture was dried (MgSO₄), filtered through celite and evaporated in vacuo. Purification by flash chromatography on silica gel (30-40% ethyl acetate/hexanes) gave 7-Hydroxy-hept-5-ynoic acid methyl ester **30** (3.022 g, 19.3 mmol, 92% from 7-(*tert*-Butyldimethyl-silanyloxy)-hept-2-yn-1-ol **27**).
**7-Iodo-hept-5-ynoic acid methyl ester (31)**. A solution of **30** (1.347 g, 8.6 mmol) in 5 mL dichloromethane was added to a mixture of triphenylphosphine (2.725 g, 10.4 mmol), imidazole (726 mg, 10.7 mmol), and iodine (2.602 g, 10.3 mmol) in 34 mL dichloromethane, rinsing with 5 mL dichloromethane. After 40 min., the dichloromethane was evaporated in vacuo to a few mL's and the resulting mixture filtered through basic alumina, washing with 10% ethyl acetate/hexanes. Purification by flash chromatography on silica gel (10% ethyl acetate/hexanes) gave 1.878 g (7.1 mmol, 83%) of the propargyl iodide.
***tert*-Butyl-{(E)-1-[2-(3-chloro-benzo[b]thiophen-2-yl)-ethyl]-3-iodo-allyloxy}-dimethyl-silane (32).** A solution of alkyne **19** (5.547 g, 15.2 mmol) in dichloromethane (50 mL) was treated with Cp₂ZrHCl (5.794 g, 22.5 mmol). The reaction was stirred for 45 min. and then N-iodosuccinimide (4.966 g, 22.1 mmol) was added. After 15 min., saturated sodium bicarbonate solution (200 mL) was added and the mixture was extracted with dichloromethane (2 x 100 mL). The combined dichloromethane solution was dried (MgSO₄), filtered and evaporated. Purification by flash chromatography on silica gel (0-5% ethyl acetate/hexanes) gave 6.608 g (13.1 mmol, 86%) of the vinyl iodide (**32**).
**7-{(1*R*,4*S*,5*R*)-4-(*tert*-Butyl-dimethyl-silanyloxy)-5-[(E)-3-(tert-butyl-dimethyl-silanyloxy)-5-(3-chloro-benzo[b]thiophen-2-yl)-pent-1-enyl]-3,3-dimethyl-2-oxo-cyclopentyl}-hept-5-ynoic acid methyl ester (33)**. A -78 °C solution of iodide **32** (675 mg, 1.34 mmol) in THF (2.0 mL) was treated with *tert*-butyllithium (1.73 mL, 2.94 mL, 1.7 M/pentane). The dark red mixture was stirred for 25 min. and then dimethylzinc (0.80 mL, 1.6 mmol, 2 M/toluene) was added. The solution was stirred at 0 °C for 15 min. and then recooled to -78 °C. At this time, a solution of enone **6** (208 mg, 0.87 mmol) in THF (1.0 mL) was added over 2 h by syringe pump, rinsing with 0.5 mL THF. After 30 min., HMPA (1.34 mL, distilled from CaH₂) was added followed by a solution of propargyl iodide **31** (1.286 g, 4.83 mmol) in THF (1.0 mL). The solution was stirred in a -40 °C bath overnight and then 20 mL saturated ammonium chloride solution and 10 mL water were added. The mixture was extracted with dichloromethane (20 mL) and ethyl acetate (2 x 20 mL). The combined organic extracts were dried (MgSO₄), filtered and evaporated. Purification by flash chromatography on silica gel (5-10% ethyl acetate/hexanes) gave 198 mg (0.27 mmol, 31 %) of 33.
**(*Z*)-7-{(1*R*,4*S*,5*R*)-5-[(*E*)-5-(3-chloro-benzo[b]thiophene-2-yl)-3-hydroxy-pent-1-enyl]-4-hydroxy-3,3-dimethyl-2-oxo-cyclopentyl}-hept-5-ynoic acid methyl ester (34, 35)**. A solution of **33** (198 mg, 0.27 mmol) in CH₃CN (6.5 mL) was treated with HF-pyridine (1.2 mL). The solution was stirred for 3 h and saturated sodium bicarbonate solution (120 mL) was added. The mixture was extracted with dichloromethane (3 x 50 mL) and the combined dichloromethane solution dried (Na₂SO₄), filtered and evaporated. Purification by flash chromatography (50% ethyl acetate/hexane) followed by preparative TLC (55% ethyl acetate/hexane) gave 55 mg (0.11 mmol, 41 %) of the less polar diastereomer (**34**) and 51 mg (0.10 mmol, 37%) of the more polar diastereomer (**35**).
**(*Z*)-7-{(1*R*,4S,5*R*)-5-[(*E*)-5-(3-chloro-benzo[b]thiophene-2-yl)-3-hydroxy-pent-1-enyl]-4-hydroxy-3,3-dimethyl-2-oxo-cyclopentyl}-hept-5-ynoic** acid **(low R_{f} diastereomer, 36).** A solution of **34** (9 mg, 0.017 mmol) and rabbit liver esterase (1 mg) in pH 7.2 phosphate buffer (2 mL)/CH₃CN (0.1 mL) was stirred for 17 h. The mixture was then coevaporated with CH₃CN to remove water and the residue purified by flash chromatography on silica gel (3-7% MeOH/CH₂Cl₂) to give 8 mg (0.016 mmol, 93%) of the acid (**36**).
**(*Z*)-7-{(1*R*,4*S*,5R)-5-[(*E*)-5-(3-chloro-benzo[b]thiophene-2-yl)-3-hydroxy-pent-1-enyl]-4-hydroxy-3,3-dimethyl-2-oxo-cyclopentyl}-hept-5-ynoic acid (high R_{f} diastereomer, 37).** A solution of **35** (12 mg, 0.023 mmol) and rabbit liver esterase (1 mg) in pH 7.2 phosphate buffer (2 mL)/CH₃CN (0.1 mL) was stirred for 17 h. TLC showed the presence of starting material, so another 2 mg of the esterase was added. After stirring for another 24 h, the reaction was complete. Work up and purification as above for **36** gave 8 mg (0.016 mmol, 69%) of the acid (**37**).
**(*Z*)-7-{(1*R*,4*S*,5*R*)-5-[(*E*)-5-(3-chloro-benzo[b]thiophene-2-yl)-3-hydroxy-pent-1-enyl]-4-hydroxy-3,3-dimethyl-2-oxo-cyclopentyl}-hept-5-enoic acid methyl ester (low R_{f} diastereomer, 38).** Ethanol (95%, 2.5 mL) was added to NiCl₂ (50 mg, 0.39 mmol) and NaBH₄ (7 mg, 0.19 mmol). The resulting black mixture was stirred for 5 min. and then ethylenediamine (41 µL, 0.61 mmol) was added. After 15 min., a solution of alkyne **34** (40 mg, 0.077 mmol) in 0.5 mL 95% ethanol was added, rinsing with 0.5 mL ethanol. The flask was purged with H₂ and allowed to stir under 1 atm H₂ for 22 h. The mixture was then filtered through celite and purified by flash chromatography on silica gel (55% ethyl acetate/hexanes) to give 17 mg (0.032 mmol, 43%) of the alkene (**38**).
**(Z)-7-{(1R,4S,5R)-5-[(E)-5-(3-chloro-benzo[b]thiophene-2-yl)-3-hydroxy-pent-1-enyl]-4-hydroxy-3,3-dimethyl-2-oxo-cyclopentyl}-hept-5-enoic acid methyl ester (high R_{f} diastereomer 39)**. The same procedure as for **36** was followed to give 17 mg (0.032 mmol, 41 %) of **39.**
**(Z)-7-{(1*R*,4*S*,S*R*)-5-[(*E*)-5-(3-chloro-benzo[b]thiophene-2-yl)-3-hydroxy-pent-1-enyl]4-hydroxy-3,3-dimethyl-2-oxo-cyclopentyl}-hept-5-enoic acid** (**low R_{f} diastereomer, 40**). The same procedure as above for **36** was used to give 9 mg (0.018 mmol, 85%) of acid **40**. 300 MHz ¹H NMR (CDCl₃, ppm) δ 7.73 (2 H, d, J = 8.4 Hz) 7.45-7.30 (2 H, m) 5.8-5.6 (2 H, m) 5.4-5.3 (2 H, m) 4.3-4.1 (1 H, m) 3.57 (1 H, d, J = 9.7 Hz) 3.1-2.9 (2 H, m) 2.5-1.9 (10 H, m) 1.7-1.6 (2 H, m) 1.09 (3 H, s) 0.89 (3 H, s).
**(Z)-7-{(1*R*,4*S*,5*R*)-5-[(*E*)-5-(3-chloro-benzo[b]thiophene-2-yl)-3-hydroxy-pent-1-enyl]-4-hydroxy-3,3-dimethyl-2-oxo-cyclopentyl}-hept-5-enoic acid (high R_{f} diastereomer, 41**). The same procedure as above for the **36** was used to give 9mg (0.018 mmol, 85%) of acid **41**. 300 MHz ¹H NMR (CDCl₃, ppm) δ 7.73 (2 H, d, J = 8.8 Hz) 7.45-7.30 (2 H, m) 5.8-5.6 (2 H, m) 5.45-5.30 (2 H, m) 4.3-4.2 (1 H, m) 3.61 (1 H, d, J = 9.7 Hz) 3.1-3.0 (2 H, m) 2.5-1.9 (10 H, m) 1.7-1.6 (2 H, m) 1.10 (3 H, s) 0.90 (3 H, s).
**(Z)-7-[(1*R*,4*S*,5*R*)-5-((*E*)-4-Benzo[b]thiophen-2-yl-3-hydroxy-but-1-enyl)-4-hydroxy-3,3-dimethyl-2-oxo-cyclopentyl]-hept-5-enoic acid (2-hydroxyethyl)-amide (65)**. A solution of acid **55** (prepared similarly to acid **41**, 7 mg, 0.015 mmol) in DMF (0.5 mL) was treated with N-hydroxysuccinimide (6.9 mg, 0.056 mmol). The mixture was stirred for 5 minutes and 1-[3-(dimethylamino)propyl]-3-ethylcarbodiimide hydrochloride (EDCI,20.7 mg, 0.11 mmol) was added. After stirring for 7 h, 2-aminoethanol (5 µL, 0.083 mmol) was added and the mixture stirred further for 16 h. Ethyl acetate (50 mL) was added and the mixture was washed with water (3 x 50 mL) and brine (50 mL). The organic layer was dried (Na₂SO₄), filtered and evaporated. Purification by flash chromatography on silica gel (5% methanol/dichloromethane) followed by preparative thin layer chromatography (10% methanol/dichloromethane) gave amide **65** (5 mg, 0.010 mmol, 65%).
**(Z)-7-[(1*R*,4*S*,5*R*)-5-((*E*)-4-Benzo[b]thiophen-2-yl-3-hydroxy-but-1-enyl)-4-hydroxy-3,3-dimethyl-2-oxo-cyclopentyl]-hept-5-enoic acid amide (69)**. A solution of acid 55 (9 mg, 0.02 mmol) in dichloromethane (0.2 mL) was treated with triethylamine (15 µL, 0.11 mmol). The solution was cooled to 0 °C and after 10 minutes, ethyl chloroformate (7 µL, 0.073 mmol) was added. The solution was stirred further for 1 h at 0 °C and then concentrated aqueous ammonium hydroxide solution was added (10 µL, 0.26 mmol). The reaction was allowed to stir at room temperature overnight and then was quenched by addition of 0.5 M HCl (7 mL). The mixture was extracted with ethyl acetate (3 x 30 mL) and the combined ethyl acetate solution was washed with saturated NaHCO₃ solution (20 mL) and brine (20 mL) and then was dried (Na₂SO₄), filtered and evaporated. Purification by flash chromatography on silica gel (2% - 6% methanol/dichloromethane) gave the title amide (2.6 mg, 28 %).

The methods of screening the compounds of this invention for the desired biological activity are illustrated in the following non-limiting examples. Results for example compounds of this invention are included in Table 2.

### Radioligand Binding

### Cells Stably Expressing EP₁, EP₂, EP₄ and FP Receptors

HEK-293 cells stably expressing the human or feline FP receptor, or EP₁, EP₂, or EP₄ receptors were washed with TME buffer, scraped from the bottom of the flasks, and homogenized for 30 sec using a Brinkman PT 10/35 polytron. TME buffer was added to achieve a final 40 ml volume in the centrifuge tubes (the composition of TME is 100 mM TRIS base, 20 mM MgCl₂, 2M EDTA; 10N HCl is added to achieve a pH of 7.4).

The cell homogenate was centrifuged at 19000 r.p.m. for 20 min at 4° C using a Beckman Ti-60 rotor. The resultant pellet was resuspended in TME buffer to give a final 1 mg/ml protein concentration, as determined by Biorad assay. Radioligand binding competition assays vs. [³H-]17 -phenyl PGF_{2α} (5 nM) were performed in a 100µl volume for 60 min. Binding reactions where started by adding plasma membrane fraction. The reaction was terminated by the addition of 4 ml ice-cold TRIS-HCl buffer and rapid filtration through glass fiber GF/B filters using a Brandel cell harvester. The filters were washed 3 times with ice-cold buffer and oven dried for one hour.

[³H-] PGE₂ (specific activity 180 Ci mmol) was used as the radioligand for EP receptors. [³H] 17-phenyl PGF_{2α} was employed for FP receptor binding studies. Binding studies employing EP₁, EP₂, EP₄ and FP receptors were performed in duplicate in at least three separate experiments. A 200µl assay volume was used. Incubations were for 60 min at 25°C and were terminated by the addition of 4 ml of ice-cold 50 mM TRIS-HCl, followed by rapid filtration through Whatman GF/B filters and three additional 4 ml washes in a cell harvester (Brandel). Competition studies were performed using a final concentration of 5 nM [³H]-PGE₂, or 5 nM [³H] 17-phenyl PGE_{2α} and nonspecific binding determined with 10⁻⁵M of unlabeled PGE₂, or 17-phenyl PGF_{2α}, according to receptor subtype studied.

### METHODS FOR FLIPR™ STUDIES

### (a) CELL CULTURE

HEK-293(EBNA) cells, stably expressing one type or subtype of recombinant human prostaglandin receptors (prostaglandin receptors expressed: hDP/Gqs5; hEP₁; hEP₂/Gqs5; hEP_{3A}/Gqi5; hEP₄/Gqs5; hFP; hIP; hTP), were cultured in 100 mm culture dishes in high-glucose DMEM medium containing 10% fetal bovine serum, 2 mM 1-glutamine, 250 µg/ml geneticin (G418) and 200 µg/ml hygromycin B as selection markers, and 100 units/ml penicillin G, 100 µg/ml streptomycin and 0.25 µg/ml amphotericin B.

### (b) CALCIUM SIGNAL STUDIES ON THE FLIPR™

Cells were seeded at a density of 5x10⁴ cells per well in Biocoat® Poly-D-lysine-coated black-wall, clear-bottom 96-well plates (Becton-Dickinson) and allowed to attach overnight in an incubator at 37 °C. Cells were then washed two times with HBSS-HEPES buffer (Hanks Balanced Salt Solution without bicarbonate and phenol red, 20 mM HEPES, pH 7.4) using a Denley Cellwash plate washer (Labsystems). After 45 minutes of dye-loading in the dark, using the calcium-sensitive dye Fluo-4 AM at a final concentration of 2 µM, plates were washed four times with HBSS-HEPES buffer to remove excess dye leaving 100 µl in each well. Plates were re-equilibrated to 37 °C for a few minutes.

Cells were excited with an Argon laser at 488 nm, and emission was measured through a 510-570 nm bandwidth emission filter (FLIPR™, Molecular Devices, Sunnyvale, CA). Drug solution was added in a 50 µl volume to each well to give the desired final concentration. The peak increase in fluorescence intensity was recorded for each well. On each plate, four wells each served as negative (HBSS-HEPES buffer) and positive controls (standard agonists: BW245C (hDP); PGE₂ (hEP₁; hEP₂/Gqs5; hEP_{3A}/Gqi5; hEP₄/Gqs5); PGF_{2α} (hFP); carbacyclin (hIP); U-46619 (hTP), depending on receptor). The peak fluorescence change in each drug-containing well was then expressed relative to the controls.

Compounds were tested in a high-throughput (HTS) or concentration-response (CoRe) format. In the HTS format, forty-four compounds per plate were examined in duplicates at a concentration of 10⁻⁵ M. To generate concentration-response curves, four compounds per plate were tested in duplicates in a concentration range between 10⁻⁵ and 10⁻¹¹ M. The duplicate values were averaged. In either, HTS or CoRe format each compound was tested on at least 3 separate plates using cells from different passages to give an n ≥ 3.

**Table 2**

| **Compound** | **hFP** | **hEP₁** | **hEP₂** | **hEP_{3D} hEP_{3A}** | **hEP4** | **hDP** | **hIP** | **hTP** |
|---|---|---|---|---|---|---|---|---|
| **21** | NA | NA | >10 K | NA | 98 | NA | NA | NA |
| **22** | | | NA | NA | 300 | | | |
| | NA | NA | NA | NA | 30 | NA | NA | NA |
| **23** | | | NA | >10K | 44 | | | |
| | NA | NA | NA | NA | 0.1 | NA | NA | >10K |
| **24** | | | NA | >>10K | 26 | | | |
| | NA | NA | NA | NA | 0.1 | NA | NA | NA |
| **34** | | | NA | | >10K | | | |
| | NA | | | NA | >10K | | NA | NA |
| **35** | NA | | | NA | 2455 | | NA | NA |
| **36** | | | NA | | 200 | | | |
| | NA | | | NA | 66 | | >10K | NA |
| **37** | | | NA | | 100 | | | |
| | NA | | | NA | 32 | | >10K | NA |
| **38** | | | NA | | 2700 | | | |
| | NA | | | NA | 269 | | NA | NA |
| **39** | | | NA | | 2300 | | | |
| | NA | | | NA | 141 | | NA | NA |
| **40** | | | NA | | 200 | | | |
| | NA | | | NA | 0.3 | | NA | >10K |
| **41** | | | >10K | | 20 | | | |
| | NA | | | NA | | | NA | >10K |
| **42** | | | NA | >10⁴ | >10⁴ | | | |
| | NA | NA | NA | 559 | NA | NA | NA | NA |
| **43** | | | NA | 1700 | 400 | | | |
| | NA | >10⁴ | NA | 11 | 63 | | 3981 | 18 |
| **44** | | | 1500 | 300 | 5.5 | | | |
| | NA | 782 | 944 | 4.6 | 0.2 | >10 K | 284 | 18 |
| **45** | | | NA | >10⁴ | 400 | | NA | 631 |
| | NA | NA | NA | 531 | 51 | NA | NA | NA |
| **46** | | | >10K | >10K | 4 | | | |
| | NA | 290 | | 589 | 0.4 | NA | NA | |
| **47** | NA | 963 | NA | >10K | 76 | | NA | |
| **48** | | | NA | | 45 | | | |
| **49** | | | NA | | 1400 | | | |
| **50** | | | NA | 6607 | 2400 | | | |
| | NA | 638 | | >10K | 3162 | | NA | >10K |
| **51** | NA | | NA | NA | 700 | | NA | |
| **52** | | | NA | | 72 | | | |
| | NA | 27 | | 60 | 18 | | NA | |
| **53** | | | | | 59 | | | |
| | NA | 1020 | NA | 1862 | 6.4 | | NA | |
| **54** | | | NA | 4700 | 20 | | | |
| | NA | 308 | NA | | 0.3 | NA | NA | |
| **55** | | | NA | >10K | 310 | | | |
| | NA | 758 | NA | | 38 | NA | NA | |
| **60** | NA | NA | NA | NA | >10K | NA | NA | NA |
| **61** | NA | NA | NA | NA | NA | NA | NA | NA |
| **62** | NA | NA | NA | NA | 832 | NA | NA | NA |
| **63** | NA | >10K | NA | NA | 478 | NA | NA | NA |
| **64** | NA | NA | NA | NA | 4154 | NA | NA | NA |
| **65** | NA | NA | NA | NA | NA | NA | NA | NA |
| **68** | NA | NA | NA | NA | 678 | NA | NA | >10K |
| **69** | NA | NA | NA | NA | 5000 | NA | NA | >10K |
| **70** | NA | NA | NA | >10K | 219 | NA | NA | |
| **71** | NA | NA | NA | NA | 10000 | NA | NA | |
| **72** | NA | NA | NA | NA | >10K | NA | NA | NA |
| **73** | NA | NA | NA | NA | NA | NA | NA | NA |
| **74** | NA | 2376 | NA | | 256 | NA | NA | |
| **75** | NA | 2050 | NA | | >10K | NA | NA | >10K |

| | | | | | | | | |
|---|---|---|---|---|---|---|---|---|
| The top numbers are the radioligand binding values(nm) The bottom numbers are the functional data (nm) | | | | | | | | |

## Claims

1. A compound represented by Formula I: 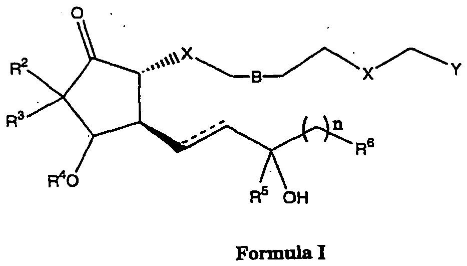 wherein the dashed lines indicate the presence or absence of a bond, the hatched wedges indicate the α (down) configuration, and the solid triangles indicate the β (up) configuration;
B is a single, double, or triple covalent bond;
n is 0-6;
X is CH₂, S or O wherein both X moieties are identical;
Y is any pharmaceutically acceptable salt of CO₂H, or CO₂R, CONR₂, CONHCH₂CH₂OH, CON(CH₂CH₂OH)₂, CH₂OR, P(O)(OR)₂, CONRSO₂R, SONR₂, or 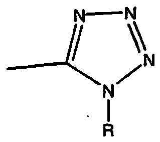 R is H, C₁₋₆ alkyl or C₂₋₆ alkenyl;
R² and R³ are C₁₋₆ linear alkyl which may be the same or different, and may be bonded to each other such that they form a ring incorporating the carbon to which they are commonly attached;
R⁴ is hydrogen, R, C(=O)R, or any group that is easily removed under physiological conditions such that R⁴ is effectively hydrogen;
R⁵ is hydrogen or R;
R⁶ is
i) hydrogen;
ii) a linear or branched hydrocarbon containing between 1 and 8 carbon atoms, which may contain one or more double or triple bonds, or oxygen or halogen derivatives of said hydrocarbon, wherein 1-3 carbon or hydrogen atoms may be substituted by O or a halogen; or
iii) aryloxy, heteroaryloxy, C₃₋₈ cycloalkoxy, C₃₋₈ cycloalkyl, C₆₋₁₀ aryl or C₃₋₁₀ heteroaryl, wherein one or more carbons is substituted with N, O, or S; and which may contain one or more substituents selected from the group consisting of halogen, trihalomethyl, cyano, nitro, amino, hydroxy, C₆₋₁₀ aryl, C₃₋₁₀ heteroaryl, aryloxy, heteroaryloxy, C₁₋₆ alkyl, OR, SR, and SO₂R; and
the compound of Formula I is not a compound of Formula II 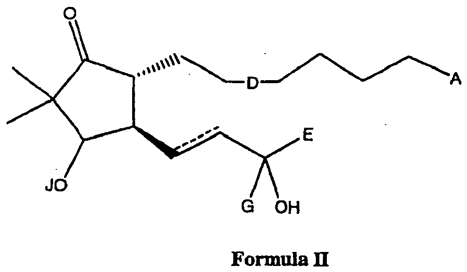 wherein A is CO₂H, CO₂Me, or CO₂Et;
D is a single, double, or triple covalent bond;
E is a linear, branched, or cycloalkyl chain of 3 to 7 carbons, trifluoromethylbutyl, hydroxylalkyl, or CH₂R⁷, wherein R⁷ is phenyl, cyclopentyl, phenoxy, chlorophenoxy, propoxy, or-CH₂SCH₂CH₃;
J is hydrogen, R, C(=O)R, or any group that is easily removed under physiological conditions such that R⁴ is effectively hydrogen; and
G is H or CH₃.

2. The compound of claim 1 which is further represented by Formula III 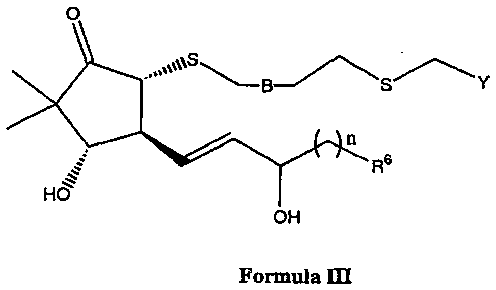 wherein Y is CO₂R, or any pharmaceutically acceptable salt of CO₂H.

3. The compound of claim 2, wherein R⁶ is C₆₋₁₀ aryl, or C₃₋₁₀ heteroaryl,
wherein one or more carbons is substituted with N, O, or S; and which may contain one or more substituents selected from the group consisting of halogen, trihalomethyl, cyano, nitro, amino, hydroxy, C₁₋₆ alkyl, OR, SR, and SO₂R.

4. The compound of claim 3, wherein R⁶ is napthyl, benzofuranyl, or benzothienyl, which may contain one or more substituents selected from the group consisting of halogen, trihalomethyl, cyano, nitro, amino, hydroxy, C₁₋₆ alkyl, OR, SR, and SO₂R.

5. The compound of claim 4, wherein Y is CO₂H or CO₂Me.

6. The compound of claim 5, where R⁶ is 3-chlorobenzothien-2-yl.

7. The compound of claim 6, where n is 2.

8. The compound of claim 7, where B is a single bond.

9. The compound of claim 1 which is further represented by Formula IV 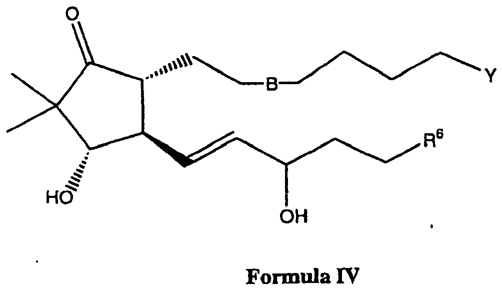 wherein Y is CO₂R or any pharmaceutically acceptable salt of CO₂H; and
R⁶ is C₆₋₁₀ aryl or C₃₋₁₀ heteroaryl, wherein one or more carbons is substituted with N, O, or S; and which may contain one or more substituents selected from the group consisting of halogen, trihalomethyl, cyano, nitro, amino, hydroxy, C₁₋₆ alkyl, OR, SR, and SO₂R.

10. The compound of claim 9, wherein Y is CO₂H or CO₂Me.

11. The compound of claim 10, wherein R⁶ is phenyl.

12. The compound of claim 11, wherein B is a double bond.

13. The compound of claim 10, wherein R⁶ is napthyl, benzofuranyl, or benzothienyl, which may contain one or more substituents selected from the group consisting of halogen, trihalomethyl, cyano, nitro, amino, hydroxy, C₁₋₆ alkyl, OR, SR, and SO₂R.

14. The compound of claim 13, wherein R⁶ is 3-chlorobenzothien-2-yl.

15. The compound of claim 14, wherein B is a double or triple bond.

16. The compound of claim 1 which is further represented by Formula V 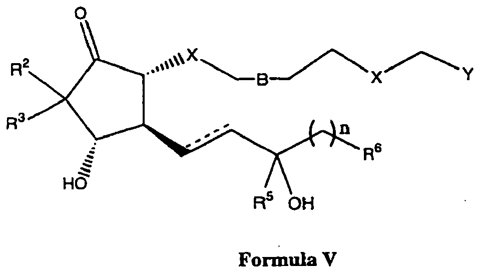 wherein at least one of R² and R³ is not methyl.

17. The compound of claim 16, wherein R² and R³ have a total number of carbon atoms of 6 or less.

18. The compound of claim 17, wherein R⁵ is hydrogen.

19. The compound of claim 1, wherein said compound is selected from the group consisting of
(3-{(1*R*,4*S*,5*S*)-5-(3-chloro-benzo[b]thiophen-2-yl)-3-hydroxy-pent-1-enyl]-4-hydroxy-3,3-dimethyl-2-oxo-cyclopentylsulfanyl}-propylsulfanyl)-acetic acid methyl ester (**21**, **22**);
(3-{(1*R*,4*S*,5*S*)-5-(3-chloro-benzo[b]thiophen-2-yl)-3-hydroxy-pent-1-enyl]-4-hydroxy-3,3-dimethyl-2-oxo-cyclopentylsulfanyl}-propylsulfanyl)-acetic acid (**23**, **24**);
(Z)-7-{(1*R*,4*S*,5*R*)-5-[(*E*)-5-(3-chloro-benzo[b]thiophene-2-yl)-3-hydroxy-pent-1-enyl]-4-hydroxy-3,3-dimethyl-2-oxo-cyclopentyl}-hept-5-ynoic acid methyl ester (**34**, **35**);
(Z)-7-{(1*R*,4*S*,5*R*)-5-[(*E*)-5-(3-chloro-benzo[b]thiophene-2-yl)-3-hydroxy-pent-1-enyl]-4-hydroxy-3,3-dimethyl-2-oxo-cyclopentyl}-hept-5-ynoic acid (**36**,**37**);
(Z)-7-{(1*R*,4*S*,5*R*)-5-[(*E*)-5-(3-chloro-benzo[b]thiophene-2-yl)-3-hydroxy-pent-1-enyl]-4-hydroxy-3,3-dimethyl-2-oxo-cyclopentyl}-hept-5-enoic acid methyl ester (**38**, **39**);
(Z)-7-{(1*R*,4*S*,5*R*)-5-[(*E*)-5-(3-chloro-benzo[b]thiophene-2-yl)-3-hydroxy-pent-1-enyl]-4-hydroxy-3,3-dimethyl-2-oxo-cyclopentyl}-hept-5-enoic acid (**40**,**41**);
(Z)-7-[(1R,4S,5R)-4-Hydroxy-5-((E)-3-hydroxy-5-phenyl-pent-1-enyl)-3,3-dimethyl-2-oxo-cyclopentyl]-hept-5-enoic acid methyl ester (**50**,**51**)
(Z)-7-[(1R,4S,5R)-4-Hydroxy-5-((E)-3-hydroxy-5-phenyl-pent-1-enyl)-3,3-dimethyl-2-oxo-cyclopentyl]-hept-5-enoic acid (**52**,**53**)
(Z)-7-[(1*R*,4*S*,5*R*)-5-((*E*)-4-Benzo[b]thiophen-2-yl-3-hydroxy-but-1-enyl)-4-hydroxy-3,3-dimethyl-2-oxo-cyclopentyl]-hept-5-enoic acid (**54**,**55**)
7-[(1*R*,4*S*,5*R*)-5-((*E*)-4-Benzo[b]thiophen-2-yl-3-hydroxy-but-1-enyl)-4-hydroxy-3,3-dimethyl-2-oxo-cyclopentyl]-heptanoic acid (**56**,**57**)
(Z)-7-[(1*R*,4*S*,5*R*)-5-(4-Benzo[b]thiophen-2-yl-3-hydroxy-butyl)-4-hydroxy-3,3-dimethyl-2-oxo-cyclopentyl]-hept-5-enoic acid (**58**,**59**)
(Z)-7-[(1*R*,4*S*,5*R*)-5-((*E*)-4-Benzo[b]thiophen-2-yl-3-hydroxy-but-1-enyl)-4-hydroxy-3,3-dimethyl-2-oxo-cyclopentyl]-hept-5-enoic acid ethylamide (**60**,**61**)
(Z)-7-[(1*R*,4*S*,5*R*)-5-((*E*)-4-Benzo[b]thiophen-2-yl-3-hydroxy-but-1-enyl)-4-hydroxy-3,3-dimethyl-2-oxo-cyclopentyl]-hept-5-enoic acid diethylamide (**62**,**63**)
(Z)-7-[(1*R*,4*S*,5*R*)-5-((*E*)-4-Benzo[b]thiophen-2-yl-3-hydroxy-but-1-enyl)-4-hydroxy-3,3-dimethyl-2-oxo-cyclopentyl]-hept-5-enoic acid (2-hydroxy-ethyl)-amide (**64**,**65**)
(3*S*,4*R*,5*R*)-4-((*E*)-4-Benzo[b]thiophen-2-yl-3-hydroxy-but-1-enyl)-3-hydroxy-2,2-dimethyl-5-[(Z)-6-(1-H-tetrazol-5-yl)-hex-2-enyl]-cyclopentanone (**66**,**67**)
(Z)-7-[(1*R*,4*S*,5*R*)-5-((*E*)-4-Benzo[b]thiophen-2-yl-3-hydroxy-but-1-enyl)-4-hydroxy-3,3-dimethyl-2-oxo-cyclopentyl]-hept-5-enoic acid amide (**68**,**69**)
(Z)-7-[(1*R*,4*S*,5*R*)-5-((*E*)-4-Benzo[b]thiophen-2-yl-3-hydroxy-but-1-enyl)-4-hydroxy-3,3-dimethyl-2-oxo-cyclopentyl]-hept-5-enoic acid methyl ester (**70**,**71**)
7-[(1*R*,4*S*,5*R*)-5-((*E*)-4-Benzo[b]thiophen-2-yl-3-hydroxy-but-1-enyl)-4-hydroxy-3,3-dimethyl-2-oxo-cyclopentyl]-hept-5-ynoic acid methyl ester (**72**,**73**)
7-[(1*R*,4*S*,5*R*)-5-((*E*)-4-Benzo[b]thiophen-2-yl-3-hydroxy-but-1-enyl)-4-hydroxy-3,3-dimethyl-2-oxo-cyclopentyl]-hept-5-ynoic acid (**74**,**75**)

20. The compound of claim 1 which is further represented by Formula XIII 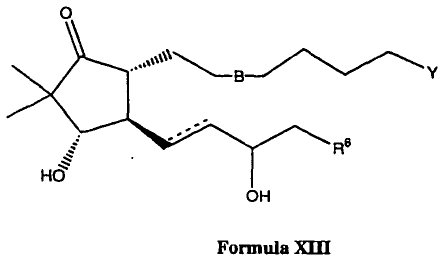 wherein B represents a single or double bond;
and R⁶ is napthyl, benzofuranyl, or benzothienyl, which may contain one or more substituents selected from the group consisting of halogen, trihalomethyl, cyano, nitro, amino, hydroxy, C₁₋₆ alkyl, OR, SR, and SO₂R.

21. The compound of claim 20, wherein R⁶ is benzotien-2-yl.

22. The compound of claim 21, wherein Y is any pharmaceutically acceptable salt of CO₂H, or CO₂R, CONR₂, CONHCH₂CH₂OH, CON(CH₂CH₂OH)₂, or

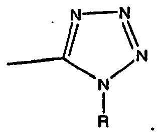

23. The compound of claim 22, wherein the dashed line indicates the presence of a bond and B is a double bond.

24. The compound of claim 22, wherein the dashed line indicates the presence of a bond and B is a single bond.

25. The compound of claim 22, wherein the dashed line indicates the absence of a bond and B is a double bond.

26. The compound of formula (I) as defined in claim 1, wherein A in Formula (II) is CO₂R⁸, wherein R⁸ is any linear, branched, or cyclic alkyl group having from 3 to 6 carbons.

27. An ophthalmic solution comprising a therapeutically effective amount of a compound as defined in any preceding claim.

28. A pharmaceutical product, comprising a container adapted to dispense the contents of said container in metered form; and an ophthalmic solution according to claim 27 in said container.

29. The method of making compounds of Formula VIII comprising: 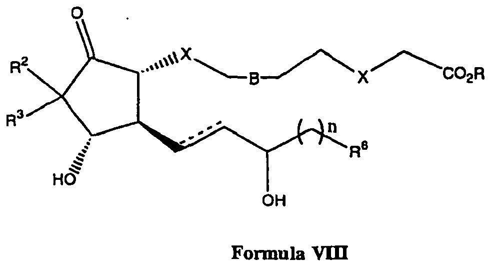
i) reacting a compound of Formula IX with a compound of Formula X in the presence of a suitable base to form a compound of formula XI; 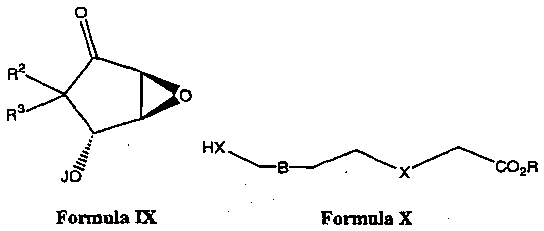 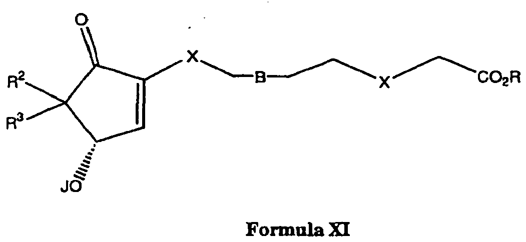
ii) coupling a compound of Formula XI with a compound of formula XII; and 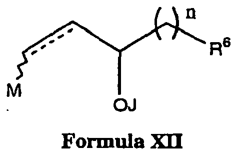
iii) removing the protecting groups and separating the diastereomers to obtain the desired products;
wherein the hatched wedges indicate the α (down) configuration, the solid triangles indicate the β (up) configuration, and the wavy lines indicate either the cis (Z) or trans (Z) conformation;
n is 0-6;
B is a single, double, or triple covalent bond;
J is a protecting group that can be easily removed to form the respective hydroxide group without affecting the rest of the molecule;
R is C₁₋₆ alkyl or C₂₋₆ alkenyl;
R² and R³ are C₁₋₆ linear alkyl which may be the same or different, and may be bonded to each other such that they form a ring incorporating the carbon to which they are commonly attached;
X is S or O wherein both X moieties are identical; and
M is a group that comprises one or more metal atoms.

30. A compound as defined in any of claims 1-26 for use in a method of treating ocular hypertension or glaucoma in a mammal.

## Patentansprüche

1. Verbindung, dargestellt durch Formel I: 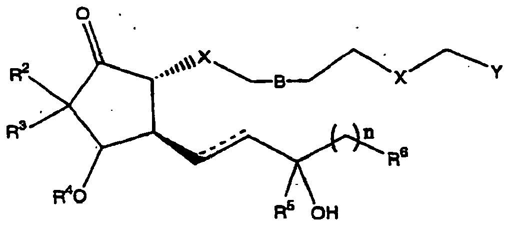 wobei gilt:
die gestrichelten Linien geben das Vorhandensein oder Nicht-Vorhandensein einer Bindung an, die straffierten Keile geben die α-Konfiguration (nach hinten) an und die durchgehenden Dreiecke geben die β-Konfiguration (nach vorne) an;
B ist eine kovalente Einfach-, Zweifach- oder Dreifachbindung;
n ist 0-6;
X ist CH₂, S oder O, wobei beide X-Reste identisch sind;
Y ist ein pharmazeutisch akzeptables Salz von CO₂H oder CO₂R, CONR₂, CONHCH₂CH₂OH, CON(CH₂CH₂OH)₂, CH₂OR, P(O)(OR)₂, CONRSO₂R, SONR₂ oder 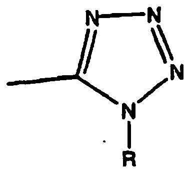
R ist H, C₁₋₆-Alkyl oder C₂₋₆-Alkenyl;
R² und R³ sind lineares C₁₋₆-Alkyl, die gleich oder verschieden sein können, und miteinander verknüpft sein können, sodass sie einen Ring bilden, wobei der Kohlenstoff, an den sie beide gebunden sind, enthalten ist;
R⁴ ist Wasserstoff, R, C(=O)R oder eine Gruppe, die unter physiologischen Bedingungen leicht entfernt wird, sodass R⁴ effektiv Wasserstoff ist;
R⁵ ist Wasserstoff oder R;
R⁶ ist
i) Wasserstoff;
ii) ein linearer oder verzweigter Kohlenwasserstoff, der zwischen 1 und 8 Kohlenstoffatome enthält, wobei eine oder mehrere Doppel- oder Dreifachbindungen enthalten sein können, oder Sauerstoff- oder Halogenderivate des genannten Kohlenwasserstoffs, wobei 1-3 Kohlenstoff-oder Wasserstoffatome durch O oder ein Halogen substituiert sein können; oder
iii) Aryloxy, Heteroaryloxy, C₃₋₈-Cycloalkoxy, C3-8-Cycloalkyl, C₆₋₁₀-Aryl oder C₃₋₁₀-Heteroaryl, wobei ein oder mehrere Kohlenstoffe mit N, O oder S substituiert ist/sind; und wobei ein oder mehrere Substituenten ausgewählt aus der Gruppe bestehend aus Halogen, Trihalomethyl, Cyano, Nitro, Amino, Hydroxy, C₆₋₁₀-Aryl, C₃₋₁₀-Heteroaryl, Aryloxy, Heteroaryloxy, C₁₋₆-Alkyl, OR, SR und SO₂R enthalten sein können; und
die Verbindung der Formel I nicht eine Verbindung der Formel II ist, 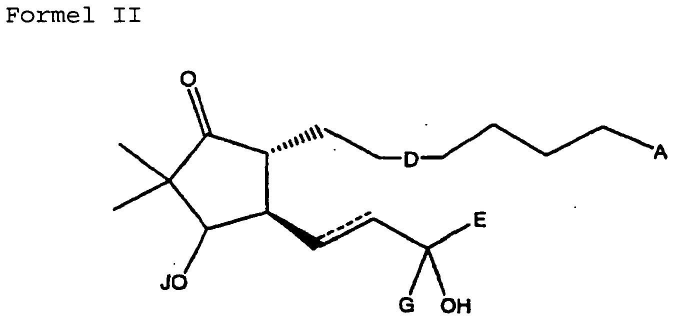 wobei gilt:
A ist CO₂H, CO₂Me oder CO₂Et;
D ist eine kovalente Einfach-, Zweifach- oder Dreifachbindung;
E ist eine lineare, verzweigte oder cyclische Alkylkette mit 3 bis 7 Kohlenstoffatomen, Trifluormethylbutyl, Hydroxyalkyl oder CH₂R⁷, wobei R⁷ Phenyl, Cyclopentyl, Phenoxy, Chlorphenoxy, Propoxy oder -CH₂SCH₂CH₃ ist;
J ist Wasserstoff, R, C(=O)R oder eine Gruppe, die unter physiologischen Bedingungen leicht entfernt wird, sodass R⁴ effektiv Wasserstoff ist; und
G ist H oder CH₃.

2. Verbindung nach Anspruch 1, die weiter durch die Formel III dargestellt wird, 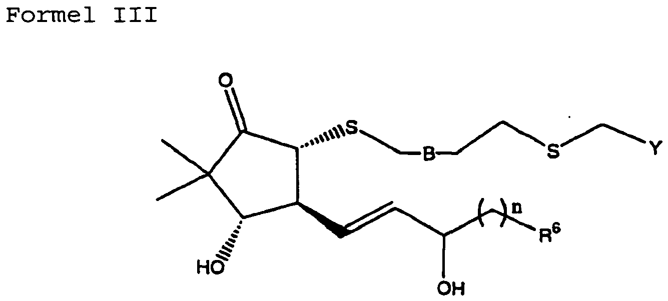 wobei Y CO₂R oder ein pharmazeutisch akzeptables Salz von CO₂H ist.

3. Verbindung nach Anspruch 2, wobei R⁶ C₆₋₁₀-Aryl oder C₃₋₁₀-Heteroaryl ist, wobei ein oder mehrere Kohlenstoffatome mit N, O oder S substituiert sind, und wobei ein oder mehrere Substituenten ausgewählt aus der Gruppe bestehend aus Halogen, Trihalomethyl, Cyano, Nitro, Amino, Hydroxy, C₁₋₆-Alkyl, OR, SR und SO₂R enthalten sein können.

4. Verbindung nach Anspruch 3, wobei R⁶ Naphthyl, Benzofuranyl oder Benzothienyl ist, die ein oder mehrere Substituenten ausgewählt aus der Gruppe bestehend aus Halogen, Trihalomethyl, Cyano, Nitro, Amino, Hydroxy, C₁₋₆-Alkyl, OR, SR und SO₂R enthalten können.

5. Verbindung nach Anspruch 4, wobei Y CO₂H oder CO₂Me ist.

6. Verbindung nach Anspruch 5, wobei R⁶ 3-Chlorbenzothien-2-yl ist.

7. Verbindung nach Anspruch 6, wobei n 2 ist.

8. Verbindung nach Anspruch 7, wobei B eine Einfachbindung ist.

9. Verbindung nach Anspruch 1, die weiter durch die Formel IV dargestellt wird, 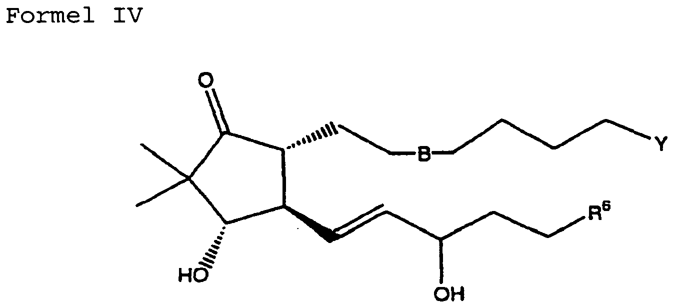 wobei gilt:
Y ist CO₂R oder ein pharmazeutisch akzeptables Salz von CO₂H ; und
R⁶ ist C₆₋₁₀-Aryl oder C₃₋₁₀-Heteroaryl, wobei ein oder mehrere Kohlenstoffatome mit N, O oder S substituiert sind; und wobei ein oder mehrere Substituenten ausgewählt aus der Gruppe bestehend aus Halogen, Trihalomethyl, Cyano, Nitro, Amino, Hydroxy, C₁₋₆-Alkyl, OR, SR und SO₂R enthalten sein können.

10. Verbindung nach Anspruch 9, wobei Y CO₂H oder CO₂Me ist.

11. Verbindung nach Anspruch 10, wobei R⁶ Phenyl ist.

12. Verbindung nach Anspruch 11, wobei B eine Doppelbindung ist.

13. Verbindung nach Anspruch 10, wobei R⁶ Naphthyl, Benzofuranyl oder Benzothienyl ist, wobei ein oder mehrere Substituenten ausgewählt aus der Gruppe bestehend aus Halogen, Trihalomethyl, Cyano, Nitro, Amino, Hydroxy, C₁₋₆-Alkyl, OR, SR und SO₂R enthalten sein können.

14. Verbindung nach Anspruch 13, wobei R⁶ 3-Chlorbenzothien-2-yl ist.

15. Verbindung nach Anspruch 14, wobei B eine Doppel- oder Dreifachbindung ist.

16. Verbindung nach Anspruch 1, die weiter durch die Formel V dargestellt wird, 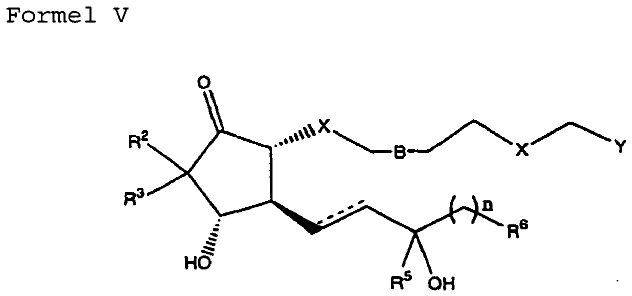 wobei wenigstens eine Gruppe von R² und R³ nicht Methyl ist.

17. Verbindung nach Anspruch 16, wobei R² und R³ insgesamt 6 oder weniger Kohlenstoffatome besitzen.

18. Verbindung nach Anspruch 17, wobei R⁵ Wasserstoff ist.

19. Verbindung nach Anspruch 1, wobei die genannte Verbindung ausgewählt ist aus der Gruppe bestehend aus
(3-{(1R,4S,5S)-5-(3-Chlor-benzo[b]thiophen-2-yl)-3-hydroxy-pent-1-enyl]-4-hydroxy-3,3-dimethyl-2-oxo-cyclopentylsulfanyl}-propylsulfanyl)-essigsäure-methylester (21,22);
(3-{(1R,4S,SS)-5-3-Chlor-benzo[b]thiophen-2-yl)-3-hydroxy-pent-1-enyl]-4-hydroxy-3,3-dimethyl-2-oxo-cyclopentylsulfanyl}-propylsulfanyl)-essigsäure (23, 24) ;
(Z)-7-{(1R,4S,5R)-5-[(E)-5-3-Chlor-benzo[b]thiophen-2-yl)-3-hydroxy-pent-1-enyl]-4-hydroxy-3,3-dimethyl-2-oxo-cyclopentyl}-hept-5-insäuremethylester (34, 35);
(Z)-7-{(1R,4S,5R)-5-[(E)-5-3-Chlor-benzo[b]thiophen-2-yl)-3-hydroxy-pent-1-enyl]-4-hydroxy-3,3-dimethyl-2-oxo-cyclopentyl}-hept-5-insäure (36, 37);
(Z)-7-{(1R,4S,5R)-5-[(E)-5-(3-Chlor-benzo[b]thiophen-2-yl)-3-hydroxy-pent-1-enyl]-4-hydroxy-3,3-dimethyl-2-oxo-cyclopentyl}-hept-5-ensäuremethylester (38,39);
(Z)-7-{(1R,4S,5R)-5-[(E)-5-(3-Chlor-benzo[b]thiophen-2-yl)-3-hydroxy-pent-1-enyl]-4-hydroxy-3,3-dimethyl-2-oxo-cyclopentyl}-hept-5-ensäure (40,41);
(Z)-7-[(1R,4S,5R)-4-Hydroxy-5-((E)-3-hydroxy-5-phenylpent-1-enyl)-3,3-dimethyl-2-oxo-cyclopentyl]-hept-5-enoic-Säuremethylester (50,51);
(Z)-7-[(1R,4S,5R)-4-Hydroxy-5-((E)-3-hydroxy-5-phenylpent-1-enyl)-3,3-dimethyl-2-oxo-cyclopentyl]-hept-5-ensäure (52, 53);
(Z)-7-[(1R,4S,5R)-5-((E)-4-Benzo[b]thiophen-2-yl-3-hydroxy-but-1-enyl)-4-hydroxy-3,3-dimethyl-2-oxo-cyclopentyl]-hept-5-ensäure (54,55);
7-[(1R,4S,5R)-5-((E)-4-Benzo[b]thiophen-2-yl-3-hydroxybut-1-enyl)-4-hydroxy-3,3-dimethyl-2-oxo-cyclopentyl]-heptansäure (56,57);
(Z)-7-[(1R,4S,5R)-5-(4-Benzo[b]thiophen-2-yl-3-hydroxybutyl)-4-hydroxy-3,3-dimethyl-2-oxo-cyclopentyl]-hept-5-ensäure (58,59);
(Z)-7-[(1R,4S,5R)-5-((E)-4-Benzo[b]thiophen-2-yl-3-hydroxy-but-1-enyl)-4-hydroxy-3,3-dimethyl-2-oxo-cyclopentyl]-hept-5-ensäure-ethylamid (60,61);
(Z)-7-[(1R,4S,5R)-5-((E)-4-Benzo[b]thiophen-2-yl-3-hydroxy-but-1-enyl)-4-hydroxy-3,3-dimethyl-2-oxo-cyclopentyl]-hept-5-ensäure-diethylamid (62,63);
(Z)-7-[(1R,4S,5R)-5-((E)-4-Benzo[b]thiophen-2-yl-3-hydroxy-but-1-enyl)-4-hydroxy-3,3-dimethyl-2-oxo-cyclopentyl]-hept-5-ensäure-(2-hydroxy-ethyl)-amid (64,65);
(3S,4R,5R)-4-((E)-4-Benzo[b]thiophen-2-yl-3-hydroxy-but-1-enyl)-3-hydroxy-2,2-dimethyl-5-[(Z)-6-(1-H-tetrazol-5-yl)-hex-2-enyl]-cyclopentanon (66,67);
(Z)-7-[(1R,4S,5R)-5-((E)-4-Benzo[b]thiophen-2-yl-3-hydroxy-but-1-enyl)-4-hydroxy-3,3-dimethyl-2-oxo-cyclopentyl]-hept-5-ensäureamid (68,69);
(Z)-7-[(1R,4S,5R)-5-((E)-4-Benzo[b]thiophen-2-yl-3-hydroxy-but-1-enyl)-4-hydroxy-3,3-dimethyl-2-oxo-cyclopentyl]-hept-5-ensäuremethylester (70,71);
7-[(1R,4S,5R)-5-((E)-4-Benzo[b]thiophen-2-yl-3-hydroxybut-1-enyl)-4-hydroxy-dimethyl-2-oxo-cyclopentyl]-hept-5-insäuremethylester (72,73);
7-[(1R,4S,5R)-5-((E)-4-Benzo[b]thiophen-2-yl-3-hydroxybut-1-enyl)-4-hydroxy-3,3-dimethyl-2-oxo-cyclopentyl]-hept-5-insäure (74,75).

20. Verbindung nach Anspruch 1, die weiter durch die Formel XIII dargestellt wird, 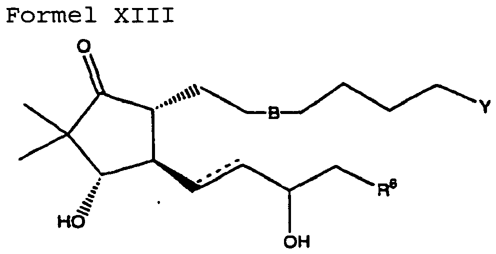 wobei gilt:
B stellt eine Einfach- oder Zweifachbindung dar;
und R⁶ ist Naphthyl, Benzofuranyl oder Benzothienyl, die ein oder mehr Substituenten ausgewählt aus der Gruppe bestehend aus Halogen, Trihalomethyl, Cyano, Nitro, Amino, Hydroxy, C₁₋₆-Alkyl, OR, SR und SO₂R enthalten können.

21. Verbindung nach Anspruch 20, wobei R⁶ Benzothien-2-yl ist.

22. Verbindung nach Anspruch 21, wobei Y ein pharmazeutisch akzeptables Salz von CO₂H oder CO₂R, CONR₂, CONHCH₂CH₂OH, CON(CH2CH₂OH)₂ oder 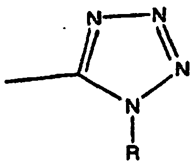 ist.

23. Verbindung nach Anspruch 22, wobei die gestrichelte Linie das Vorhandensein einer Bindung angibt und B eine Doppelbindung ist.

24. Verbindung nach Anspruch 22, wobei die gestrichelte Linie das Vorhandensein einer Bindung angibt und B eine Einfachbindung ist.

25. Verbindung nach Anspruch 22, wobei die gestrichelte Linie das Nicht-Vorhandensein einer Bindung angibt und B eine Doppelbindung ist.

26. Verbindung der Formel (I) nach Anspruch 1, wobei A in Formel (II) CO₂R⁸ ist, wobei R⁸ eine lineare, verzweigte oder cyclische Alkylgruppe mit 3 bis 6 Kohlenstoffatomen ist.

27. Ophthalmische Lösung, umfassend eine therapeutisch wirksame Menge einer Verbindung nach einem der vorhergehenden Ansprüche.

28. Pharmazeutikum, umfassend einen Behälter, der geeignet ist, den Inhalt des genannten Behälters in dosierter Form abzugeben; und eine ophthalmische Lösung nach Anspruch 27 in dem genannten Behälter.

29. Verfahren zur Herstellung der Verbindungen der Formel VIII, umfassend: 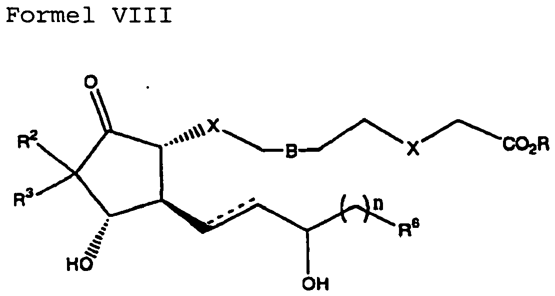
i) Umsetzen einer Verbindung der Formel IX mit einer Verbindung der Formel X in der Gegenwart einer geeigneten Base, um eine Verbindung der Formel XI zu bilden; 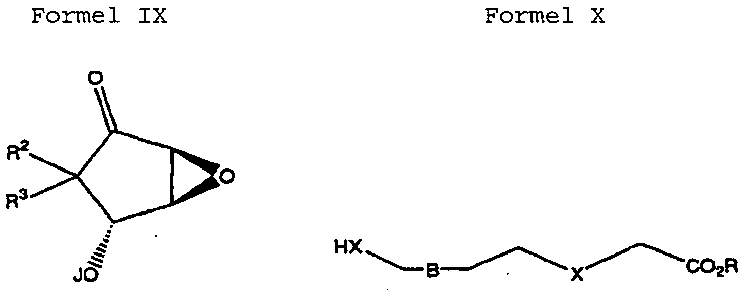 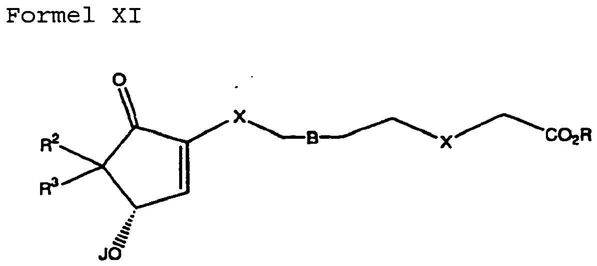
ii) Kuppeln einer Verbindung der Formel XI mit einer Verbindung der Formel XII; und 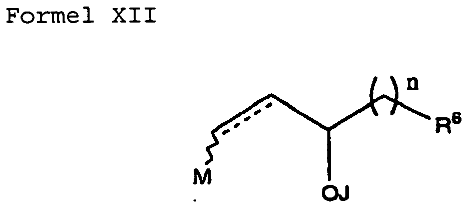
iii) Entfernen der Schutzgruppen und Trennen der Diastereomeren, um die gewünschten Produkte zu gewinnen;
wobei die schraffierten Keile die α-Konfiguration (nach hinten), die durchgehenden Dreiecke die β-Konfiguration (nach vorne) angeben und die Wellenlinien entweder die cis (Z) oder trans (Z) Konformation angeben;
n ist 0-6;
B ist eine kovalente Einfach-, Zweifach- oder Dreifachbindung;
J ist eine Schutzgruppe, die leicht entfernt werden kann, um die entsprechende Hydroxidgruppe zu bilden, ohne den Rest des Moleküls anzugreifen;
R ist C₁₋₆-Alkyl oder C₂₋₆-Alkenyl;
R² und R³ sind lineares C₁₋₆-Alkyl, die gleich oder verschieden sein können, und miteinander verknüpft sein können, sodass sie einen Ring bilden, wobei der Kohlenstoff, an den sie beide gebunden sind, enthalten ist;
X ist S oder O, wobei beide X-Reste identisch sind; und
M ist eine Gruppe, die ein oder mehrere Metallatome umfasst.

30. Verbindung nach einem der Ansprüche 1 bis 26 zur Anwendung bei einem Verfahren zur Behandlung von okularer Hypertension oder Glaukom bei einem Säugetier.

## Revendications

1. Composé représenté par la formule 1 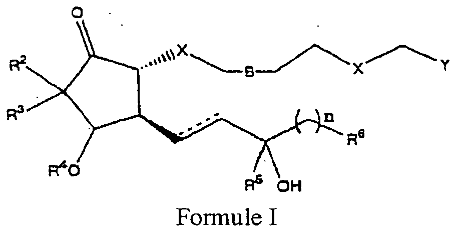 où les lignes pointillées indiquent la présence ou l'absence d'une liaison, les coins hachurés indiquent la configuration α (au-dessous), et les triangles pleins indiquent la configuration β (au-dessus);
B est une liaison covalente simple, double ou triple;
n est 0-6;
X est CH₂, S ou O, les deux groupes X étant identiques;
Y est un sel pharmaceutiquement acceptable quelconque de CO₂H, ou CO₂R, CONR₂, CONHCH₂CH₂OH, CON(CH₂CH₂OH)₂, CH₂OR, P(O)(OR)₂, CONRSO₂R, SONR₂ ou 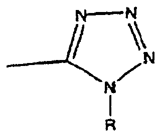
R est H, alkyle en C₁-C₆ ou alcényle en C₂-C₆;
R² et R³ sont des groupes alkyle linéaire en C₁-C₆ qui peuvent être identiques ou différents, et peuvent être liés entre eux de façon à former un cycle incorporant le carbone auquel ils sont liés tous les deux;
R⁴ est un hydrogène, R, C(=O)R, ou tout groupe qui se sépare facilement dans des conditions physiologiques de manière que R⁴ soit effectivement un hydrogène;
R⁵ est un hydrogène ou R;
R⁶ est
i) un hydrogène;
ii) un hydrocarbure linéaire ou ramifié contenant entre 1 et 8 atomes de carbone, qui peut contenir une ou plusieurs liaisons doubles ou triples, ou des dérivés oxygénés ou halogénés dudit hydrocarbure, où 1-3 atomes de carbone ou d'hydrogène peuvent être remplacés par O ou un halogène; ou
iii) aryloxy, hétéroaryloxy, cycloalcoxy en C₃-C₈, cycloalkyle en C₃-C₈, aryle en C₆-C₁₀ ou hétéroaryle en C₃-C₁₀ dans lequel un ou plusieurs atomes de carbone sont remplacés par N, O ou S; et qui peut contenir un ou plusieurs substituants choisis dans le groupe constitué par halogène, trihalogénométhyle, cyano, nitro, amino, hydroxy, aryle en C₆-C₁₀, hétéroaryle en C₃-C₁₀, aryloxy, hétéroaryloxy, alkyle en C₁-C₆, OR, SR et SO₂R; et
le composé de formule I n'est pas un composé de formule II 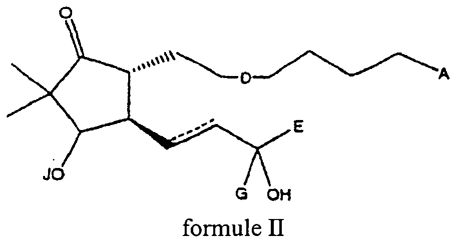 où
A est CO₂H, CO₂Me, ou CO₂Et;
D est une liaison covalente simple, double ou triple;
E est une chaîne linéaire, ramifiée ou cycloalkyle de 3 à 7 carbones, trifluorométhylbutyle, hydroxyalkyle, ou CH₂R⁷, où R⁷ est phényle, cyclopentyle, phénoxy, chlorophénoxy, propoxy ou -CH₂SCH₂CH₃;
J est un hydrogène, R, C(=O)R, ou tout groupe qui se sépare facilement dans des conditions physiologiques de manière que R⁴ soit effectivement un hydrogène;
G est H ou CH₃.

2. Composé selon la revendication 1, qui est représenté en outre par la formule III 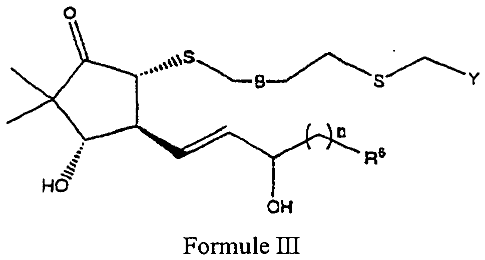 où Y est CO₂R ou tout sel pharmaceutiquement acceptable de CO₂H.

3. Composé selon la revendication 2, où R⁶ est aryle en C₆-C₁₀ ou hétéroaryle en C₃-C₁₀, où un ou plusieurs carbones sont remplacés par N, O ou S; et qui peut contenir un ou plusieurs substituants choisis dans le groupe constitué par halogène, trihalogénométhyle, cyano, nitro, amino, hydroxy, alkyle en C₁-C₆, OR, SR et SO₂R.

4. Composé selon la revendication 3, où R⁶ est naphtyle, benzofuranyle ou benzothiényle, qui peut contenir un ou plusieurs substituants choisis dans le groupe constitué par halogène, trihalogénométhyle, cyano, nitro, amino, hydroxy, alkyle en C₁-C₆, OR, SR et SO₂R.

5. Composé selon la revendication 4, où Y est CO₂H ou CO₂Me.

6. Composé selon la revendication 5, où R⁶ est 3-chlorobenzothién-2-yle.

7. Composé selon la revendication 6, où n est 2.

8. Composé selon la revendication 7, où B est une liaison simple.

9. Composé selon la revendication 1, qui est en outre représenté par la formule IV 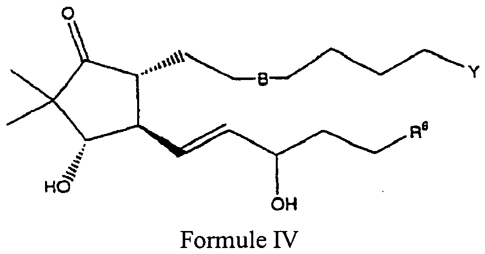 où Y est CO₂R ou tout sel pharmaceutiquement acceptable de CO₂H; et
R⁶ est aryle en C₆-C₁₀ ou hétéroaryle en C₃-C₁₀, où un ou plusieurs carbones sont remplacés par N, O ou S; et qui peut contenir un ou plusieurs substituants choisis dans le groupe constitué par halogène, trihalogénométhyle, cyano, nitro, amino, hydroxy, alkyle en C₁-C₆, OR, SR et SO₂R.

10. Composé selon la revendication 9, où Y est CO₂H ou CO₂Me.

11. Composé selon la revendication 10, où R⁶ est phényle.

12. Composé selon la revendication 11, où B est une liaison double.

13. Composé selon la revendication 10, où R⁶ est naphtyle, benzofuranyle ou benzothiényle, qui peut contenir un ou plusieurs substituants choisis dans le groupe constitué par halogène, trihalogénométhyle, cyano, nitro, amino, hydroxy, alkyle en C₁-C₆, OR, SR et SO₂R.

14. Composé selon la revendication 13, où R⁶ est 3-chlorobenzothién-2-yle.

15. Composé selon la revendication 14, où B est une liaison double ou triple.

16. Composé selon la revendication 1, qui est en outre représenté par la formule V 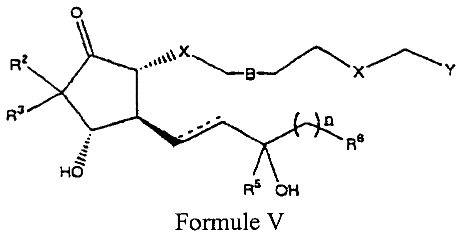 où au moins l'un des R² et R³ n'est pas un méthyle.

17. Composé selon la revendication 16, où R² et R³ ont un nombre total d'atomes de carbone d'au plus 6.

18. Composé selon la revendication 17, où R⁵ est un hydrogène.

19. Composé selon la revendication 1, ledit composé étant choisi dans le groupe constitué par:
le (3-{(1R,4S,SS)-[S-(3-chlorobenzo[b]thiophén-2-yl)-3-hydroxypent-1-ényl]-4-hydroxy-3,3-diméthyl-2-oxocyclopentylsulfanyl}propylsulfanyl)acétate de méthyle (**21**, **22**);
l'acide (3-{(1R,4S,SS)-[5-(3-chlorobenzo[b]thiophén-2-yl)-3-hydroxypent-1-ényl]-4-hydroxy-3,3-diméthyl-2-oxocyclopentylsulfanyl}propylsulfanyl)acétique (**23**, **24**);
le (Z)-7-{(1R,4S,5R)-5-[(E)-5-(3-chlorobenzo[b]thiophén-2-yl)-3-hydroxypent-1-ényl]-4-hydroxy-3,3-diméthyl-2-oxocyclopentyl}hept-5-ynoate de méthyle (**34**, **35**);
l'acide (Z)-7-{(1R,4S,SR)-5-[(E)-S-(3-chlorobenzo[b]thiophén-2-yl)-3-hydroxypent-1-ényl]-4-hydroxy-3,3-diméthyl-2-oxocyclopentyl}hept-5-ynoïque (**36**, **37**);
le (Z)-7-{(1R,4S,SR)-5-[(E)-5-(3-chlorobenzo[b]thiophén-2-yl)-3-hydroxypent-1-ényl]-4-hydroxy-3,3-diméthyl-2-oxocyclopentyl}hept-5-énoate de méthyle (**38**, **39**);
l'acide (Z)-7-{(1R,4S,5R)-5-[(E)-5-(3-chlorobenzo[b]thiophén-2-yl)-3-hydroxypent-1-ényl]-4-hydroxy-3,3-diméthyl-2-oxocyclopentyl}hept-5-énoïque (**40**, **41**);
le (Z)-7-[(1R,4S,5R)-4-hydroxy-5-((E)-3-hydroxy-5-phénylpent-1-ényl)-3,3-diméthyl-2-oxocyclopentyl]hept-5-énoate de méthyle (**50**, **51**);
l'acide (Z)-7-[(1R,4S,5R)-4-hydroxy-5-((E)-3-hydroxy-5-phénylpent-1-ényl)-3,3-diméthyl-2-oxocyclopentyl]hept-5-énoïque (**52**, **53**);
l'acide (Z)-7-[(1R,4S,5R)-5-((E)-4-benzo[b]thiophén-2-yl-3-hydroxybut-1-ényl)-4-hydroxy-3,3-diméthyl-2-oxocyclopentyl]hept-5-énoïque (**54**, **55**);
l'acide 7-[(1R,4S,5R)-5-((E)-4-benzo[b]thiophén-2-yl-3-hydroxybut-1-ényl)-4-hydroxy-3,3-diméthyl-2-oxocyclopentyl]heptanoïque (**56**, **57**);
l'acide (Z)-7-[(1R,4S,5R)-5-(4-benzo[b]thiophén-2-yl-3-hydroxybutyl)-4-hydroxy-3,3-diméthyl-2-oxocyclopentyl]hept-5-énoïque (**58**, **59**);
l'éthylamide de l'acide (Z)-7-[(1R,4S,5R)-5-((E)-4-benzo[b]thiophén-2-yl-3-hydroxybut-1-ényl)-4-hydroxy-3,3-diméthyl-2-oxocyclopentyl]hept-5-énoïque (**60**, **61**);
le diéthylamide de l'acide (Z)-7-[(1R,4S,5R)-5-((E)-4-benzo[b]thiophén-2-yl-3-hydroxybut-1-ényl)-4-hydroxy-3,3-diméthyl-2-oxocyclopentyl]hept-5-énoïque (**62**, **63**);
le (2-hydroxyéthyl)amide de l'acide (Z)-7-[(1R,4S,5R)--5-((E)-4-benzo[b]thiophén-2-yl-3-hydroxybut-1-ényl]-4-hydroxy-3,3-diméthyl-2-oxocyclopentyl]hept-5-énoïque (**64**, **65**);
la (3S,4R,5R)-4-((E)-4-benzo[b]thiophén-2-yl-3-hydroxybut-1-ényl)-3-hydroxy-2,2-diméthyl-5-(Z)-6-(1H-tétrazol-5-yl)hex-2-ényl]cyclopentanone (**66**, **67**);
l'amide de l'acide (Z)-7-[(1R,4S,5R)-5-((E)-4-benzo[b]thiophén-2-yl-3-hydroxybut-1-ényl)-4-hydroxy-3,3-diméthyl-2-oxocyclopentyl]hept-5-énoïque (**68**, **69**);
le (Z)-7-[(1R,4S,5R)-5-((E)-4-benzo[b]thiophén-2-yl-3-hydroxybut-1-ényl)-4-hydroxy-3,3-diméthyl-2-oxocyclopentyl]hept-5-énoate de méthyle (**70**, **71**);
le 7-[(1R,4S,5R)-5-((E)-4-benzo[b]thiophén-2-yl-3-hydroxybut-1-ényl)-4-hydroxy-3,3-diméthyl=2-oxocyclopentyl]hept-5-ynoate de méthyle (**72**, **73**);
l'acide 7-[(1R,4S,5R)-5-((E)-4-benzo[b]thiophén-2-yl-3-hydroxybut-1-ényl)-4-hydroxy-3,3-diméthyl-2-oxocyclopentyl]hept-5-ynoïque (**74**, **75**).

20. Composé selon la revendication 1, qui est en outre représenté par la formule XIII 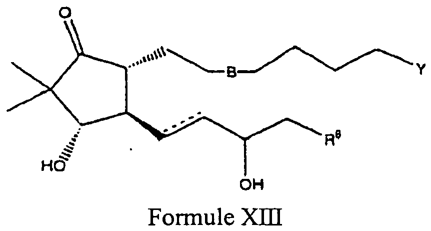 où B représente une liaison simple ou double;
et R⁶ est naphtyle, benzofuranyle ou benzothiényle, qui peut contenir un ou plusieurs substituants choisis dans le groupe constitué par halogène, trihalogénométhyle, cyano, nitro, amino, hydroxy, alkyle en C₁-C₆, OR, SR et SO₂R.

21. Composé selon la revendication 20, où R⁶ est benzothién-2-yle.

22. Composé selon la revendication 21, où Y est un sel pharmaceutiquement acceptable quelconque de CO₂H, ou CO₂R, CONR₂, CONHCH₂CH₂OH, CON(CH₂CH₂OH)₂, ou

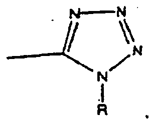

23. Composé selon la revendication 22, où la ligne pointillée indique la présence d'une liaison et B est une double liaison.

24. Composé selon la revendication 22, où la ligne pointillée indique la présence d'une liaison et B est une liaison simple.

25. Composé selon la revendication 22, où la ligne pointillée indique l'absence de liaison et B est une double liaison.

26. Composé de formule (I) tel que défini dans la revendication 1, où, dans la formule (II), A est CO₂R⁸, où R⁸ est un groupe alkyle linéaire, ramifié ou cyclique quelconque ayant de 3 à 6 atomes de carbone.

27. Solution ophtalmique comprenant une quantité thérapeutiquement efficace d'un composé tel que défini dans l'une quelconque des revendications précédentes.

28. Produit pharmaceutique, comprenant un récipient adapté pour la distribution du contenu dudit récipient sous forme dosée; et une solution ophtalmique selon la revendication 27 dans ledit récipient.

29. Procédé de préparation de composés de formule VIII, comprenant 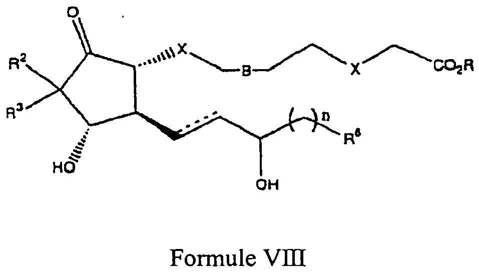
i) la réaction d'un composé de formule IX avec un composé de formule X en présence d'une base appropriée pour former un composé de formule XI; 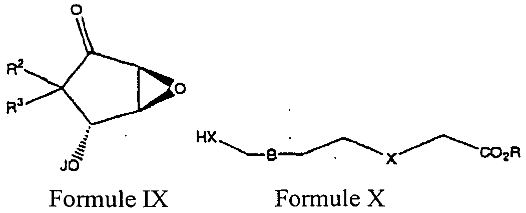 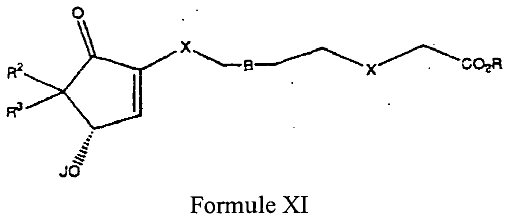
ii) le couplage d'un composé de formule XI avec un composé de formule XII; et 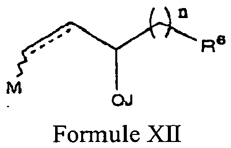
iii) l'élimination des groupes protecteurs et la séparation des diastéréoisomères pour l'obtention des produits désirés;
où les coins hachurés indiquent la configuration α (au-dessous), les triangles pleins indiquent la configuration β (au-dessus), et les lignes ondulées indiquent soit la conformation cis (Z), soit la conformation trans (Z);
n est 0-6;
B est une liaison covalente simple, double ou triple;
J est un groupe protecteur qui peut se séparer facilement pour former le groupe hydroxy correspondant sans affecter le reste de la molécule;
R est alkyle en C₁-C₆ ou alcényle en C₂-C₆;
R² et R³ sont des groupes alkyle linéaire en C₁-C₆ qui peuvent être identiques ou différents, et peuvent être liés entre eux de façon à former un cycle incorporant le carbone auquel ils sont liés tous les deux;
X est S ou O, les deux groupes X étant identiques;
M est un groupe qui comprend un ou plusieurs atomes métalliques.

30. Composé tel que défini dans l'une quelconque des revendications 1-26, pour son utilisation dans un procédé de traitement de l'hypertension oculaire ou du glaucome chez un mammifère.
